# EUROPÄISCHE PATENTANMELDUNG

(11) **EP 3 530 292 A1**
(43) Veröffentlichungstag der Anmeldung: **28.08.2019**
(21) Anmeldenummer: 18405007.8
(22) Anmeldetag: 27.02.2018
(51) Int. Cl.: A61L 2/22, A61L 2/24

(54) **ANORDNUNG ZUM EINBRINGEN VON DEKONTAMINATIONSMITTEL IN EIN CONTAINMENT**

(71) Anmelder: SKAN AG, 4123 Allschwil (CH)
(72) Erfinder: MOES, Jean-Louis, 4123 Allschwil (CH); SIGWARTH, Volker, 4334 Sisseln (CH)
(74) Vertreter: Ullrich, Gerhard

(57) **Zusammenfassung**

Die Anordnung zum Einbringen einer Sollmenge an Dekontaminationsmittel in ein Containment (**9**) umfasst zunächst einen Tank (**T**) als Vorratsbehälter zur Bereitstellung des Dekontaminationsmittels in flüssiger Form. Wesensbestandteil ist eine Dosiervorrichtung (**1**) mit einer in das Containment (**9**) gerichteten Sprühdüse (**18**) zur Verneblung des Dekontaminationsmittels. Zumindest eine Zuführung aus der Umgebungsluft (**U**), ein Druckluftanschluss (**+P**) sowie eine Steuereinrichtung (8) sind zum Betrieb der Dosiervorrichtung (**1**) vorgesehen. Die Dosiervorrichtung (**1**) hat ein Dosierbehältnis (**2**), das einen Speicherraum (**20**) mit definiertem Volumen zur Aufnahme einer einzelnen Portion von Dekontaminationsmittel besitzt. Der Speicherraum (**20**) ist zur taktweisen Füllung mit einer Anzahl (**n**) von Portionen an Dekontaminationsmittel aus dem Tank (**T**) bestimmt, und die im Speicherraum (**20**) jeweils enthaltene Portion wird durch die Sprühdüse (**18**) in das Containment (**9**) vor Aufnahme einer nächsten Portion eingebracht. Hierbei ist die Portionsanzahl (**n**) zum Erreichen der Sollmenge an benötigtem Dekontaminationsmittel gleich oder grösser 1. Der Speicherraum (**20**) ist mit einer feststehenden oder einstellbaren Grösse ausgebildet und als separater Container, Zylinder, Aussparung im Dosierbehältnis (**2**) oder als gestreckte oder geschleifte Rohrlänge beschaffen. Der Speicherraum (**20**) hat vorzugsweise ein Volumen im Bereich von 1 cm³ bis 5 cm³.

## Beschreibung

### Anwendungsgebiet der Erfindung

Die Erfindung betrifft eine Anordnung zum Einbringen einer Sollmenge an Dekontaminationsmittel in ein Containment. Als Containments gelten insbesondere Isolatoren, z.B. für die pharmazeutisch-chemische Industrie, Schleusen und Sicherheitswerkbänke, z.B. für mikrobiologische Arbeiten oder Arbeiten mit toxischen Stoffen. Der Begriff umfasst ferner alle Typen von RABS (Restricted Access Barrier System), einschliesslich mobiler und stationärer Art, wie Mittel zum Transport und Räume zur Behandlung, Isolation und/oder Diagnostik von Patienten, sowie Produktionsräume und Laboratorien. Zur Anordnung gehören ein Tank als Vorratsbehälter zur Bereitstellung des Dekontaminationsmittels in flüssiger Form sowie eine Dosiervorrichtung mit einer in das Containment gerichteten Sprühdüse zur Verneblung des Dekontaminationsmittels. Zum Betrieb der Dosiervorrichtung sind ein Druckluftanschluss sowie eine Steuereinrichtung vorgesehen.

### Stand der Technik

Aus der CH 689 178 A5 ist eine Vorrichtung zur gasförmigen Dekontamination von Reinräumen mit einer Verdampfereinheit, einem Behälter zur Bevorratung von flüssigem Dekontaminationsmittel, einer Fördereinrichtung und einer Steuereinrichtung für den Prozessablauf bekannt. Vom ausserhalb des zu dekontaminierenden Reinraums positionierten Vorratsbehälter erstreckt sich eine Schlauchleitung, während die Verdampfereinheit innerhalb des Reinraums angeordnet ist.

In der CH 699 032 B1 wird ein Verfahren zur Dekontamination eines Reinraums und von temporär darin einbringbarem Behandlungsgut offenbart. Ein im Normalzustand flüssiges Dekontaminationsmittel wird aus einem Vorratsbehälter über eine Vorlaufleitung einem beheizbaren Verdampfer zugeführt. Das im Verdampfer erzeugte dampfförmige Dekontaminationsmittel wird über eine Zuleitung allein mittels adiabatischer Expansion direkt in den Reinraum eingeleitet, um sich als Kondensat im Reinraum und bei Vorhandensein auf dem in den Reinraum eingebrachten Behandlungsgut abzusetzen. Nach definierter Einwirkzeit wird das abgesetzte Kondensat aus dem Reinraum in einer Spülphase entfernt.

Gegenstand der WO 2008/116 341 A2 ist eine Dekontaminationsanordnung für einen Reinraum innerhalb eines Isolators oder einer Schleuse und für temporär in den Reinraum einbringbares Behandlungsgut. Ein Vorratsbehälter dient zur Bereitstellung eines im Normalzustand flüssigen Dekontaminationsmittels. Vorgesehen ist ferner eine Verdampfervorrichtung mit einem beheizbaren Verdampfer, der eine Verdampferzelle besitzt. Eine erste Vorlaufleitung führt vom Vorratsbehälter zur Verdampferzelle. Ein in der ersten Vorlaufleitung angeordnetes Förderaggregat ist zum Transport des Dekontaminationsmittels in die Verdampferzelle bestimmt. Von einer Drucklufteinheit führt eine zweite Vorlaufleitung in die Verdampferzelle. Mittels einer sich von der Verdampferzelle in den Reinraum erstreckenden Strömungsverbindung erfolgt die Einleitung des in der Verdampferzelle erzeugten dampfförmigen Dekontaminationsmittels. Die Strömungsverbindung wird von einer an die Verdampferzelle angeschlossenen Düse mit deren inneren Hohlraum und der davon sich fortsetzenden Mündung gebildet. Die Düse besitzt einen Kopf, aus welchem die Mündung austritt, und hat einen Schaft, der den Boden des Reinraums hin zur Verdampferzelle durchragt.

Schliesslich wird in der WO 2013/003 967 A1 eine Vorrichtung zur Dekontamination für ein Containment und/oder von temporär darin einbringbarem Behandlungsgut vorgeschlagen, die ein Reservoir zur Bevorratung eines im Normalzustand flüssigen Dekontaminationsmittels besitzt. Ferner umfasst die Vorrichtung einen aus einer Druckluftquelle beaufschlagten Zerstäuber zur Überführung des Dekontaminationsmittels in ein Aerosol. Die Vorrichtung hat zumindest einen Auslass, der für die direkte Einleitung des in der Vorrichtung erzeugten Aerosols in das Containment vorgesehen ist. Reservoir und Zerstäuber sind integraler Bestandteil der Vorrichtung, die sich als Ganzes am oder im Containment installieren lässt. Das Reservoir ist fabrikmässig mit Dekontaminationsmittel gefüllt oder lässt sich vor dem Gebrauch vom Anwender auffüllen. Die gesamte Vorrichtung oder zumindest das Reservoir ist als Einwegartikel ausgebildet. Der Zerstäuber ist eine Venturidüse, an der ein Primärkanal mündet, der zum Reservoir führt. Am Zerstäuber mündet ein Sekundärkanal, der einen Anschluss zur Druckluftquelle hat. Die Füllmenge im Reservoir ist für ein definiertes Volumen eines Containments bemessen.

### Aufgabe der Erfindung

Bei den bisher bekannten apparativen Aufbauten mit einer Dosiervorrichtung zum Einbringen von Dekontaminationsmittel in ein Containment sind die beheizten Verdampfer oftmals nicht unproblematisch. Eine möglichst genaue Dosiermenge in das Containment einzubringen, erfordert den Einsatz einer kostenintensiven Messapparatur, zumeist eine Waage mit einsprechendem Platzbedarf und Leitungsverbindungen. Ein weiterer Nachteil vieler für den genannten Zweck vorhandener Vorrichtungen ist der erhebliche Zeitbedarf für die Durchführung eines Dekontaminationsprozesses.

In Relation zum vorbekannten Stand der Technik liegt der Erfindung die Aufgabe zugrunde, eine innovative Anordnung zum Einbringen einer Sollmenge an Dekontaminationsmittel in ein Containment zu schaffen. Damit soll hinsichtlich apparativem Aufwand, Raumbedarf, Dosiergenauigkeit, Sicherheit, breitem Anwendungsfeld und Zeitgewinn bei der Durchführung von Dekontaminationen eine gesamthaft kosteneffiziente Lösung erzielt werden.

### Übersicht über die Erfindung

Die Anordnung ist zum Einbringen einer Sollmenge an Dekontaminationsmittel in ein Containment bestimmt. Zur Anordnung gehören ein Tank als Vorratsbehälter zur Bereitstellung des Dekontaminationsmittels in flüssiger Form sowie eine Dosiervorrichtung mit einer in das Containment gerichteten Sprühdüse zur Verneblung des Dekontaminationsmittels. Zum Betrieb der Dosiervorrichtung sind ein Druckluftanschluss sowie eine Steuereinrichtung vorhanden. Die Dosiervorrichtung hat ein Dosierbehältnis, das einen Speicherraum mit definiertem Volumen zur Aufnahme einer einzelnen Portion von Dekontaminationsmittel besitzt. Der Speicherraum dient zur taktweisen Füllung mit einer Anzahl von Portionen an Dekontaminationsmittel aus dem Tank, während die im Speicherraum jeweils enthaltene Portion zum Einbringen durch die Sprühdüse in das Containment vor Aufnahme einer nächsten Portion vorgesehen ist. Die Portionsanzahl zum Erreichen der Sollmenge an benötigtem Dekontaminationsmittel ist gleich oder grösser 1 wählbar.

Nachfolgend werden spezielle Ausführungsformen der Erfindung definiert: Der Speicherraum ist mit einer feststehenden oder einstellbaren Grösse ausgebildet und als separater Container, Zylinder, Aussparung im Dosierbehältnis oder als gestreckte oder geschleifte Rohrlänge beschaffen. Der Speicherraum hat ein Volumen im Bereich von 1 cm³ bis 50 cm³, vorzugsweise im Bereich von 1 cm³ bis 5 cm³.

Zur Einstellbarkeit der Grösse der im Speicherraum aufnehmbaren Portion an Dekontaminationsmittel nutzt man beispielsweise ein Standrohr, einen Kolben oder einen Elektrofühler, die in den Speicherraum einschiebbar und in der Position verstellbar sind, oder ein Schlauch- oder Rohrwickel von bestimmtem Innenquerschnitt und Länge des Wickels.

Der Druckluftanschluss dient zur Füllung des Speicherraums mit Dekontaminationsmittel aus dem Tank und zum Betrieb der Sprühdüse, basierend auf dem Venturi-Prinzip. Zur Füllung des Speicherraums mit Dekontaminationsmittel aus dem Tank weist die Dosiervorrichtung einen Förderer auf. Zur Signalisierung einer in den Speicherraum komplett eingefüllten Portion an Dekontaminationsmittel und zum Stoppen der Förderung aus dem Tank dient ein Füllstands-Sensor, ein Verschlusselement oder ein verstellbares Standrohr, ein verstellbarer Kolben oder ein verstellbarer Elektrofühler. Das Verschlusselement ist als im Speicherraum angeordneter Auftriebskörper oder als semipermeable Membran beschaffen. Der verstellbare Elektrofühler wirkt mit einem feststehenden Elektrokontakt zusammen, welche bei komplett eingefüllter Portion an Dekontaminationsmittel durch dieses überbrückt sind.

An der Steuereinrichtung sind der Zeitablauf mit Start, Prozessverlauf und Beendigung für das Einbringen der Sollmenge an Dekontaminationsmittel in das Containment und die Sollmenge durch Bestimmung der Portionsanzahl programmierbar. Nach Beendigung des Einbringens der Sollmenge an Dekontaminationsmittel in das Containment ist auch die Rückführung des in der Dosiervorrichtung verbliebenen Dekontaminationsmittels in den Tank programmierbar.

Für das Einbringen der Sollmenge an Dekontaminationsmittel in das Containment sind zur Schaltung des Prozessverlaufs und Mengenregulierung vorgesehen:
a) eine von der Steuereinrichtung (über Steuerleitungen beaufschlagte erste Kategorie von Stellgliedern in Gestalt von 3-Wege-Ventilen, die in Dekontaminationsmittel oder Umgebungsluft führenden Stoffleitungen installiert sind;
b) eine von der Steuereinrichtung über Steuerleitungen beaufschlagte zweite Kategorie von Stellgliedern in Gestalt von Absperrventilen, die in Dekontaminationsmittel oder Druckluft führenden Stoffleitungen installiert sind; und
c) eine dritte Kategorie von Stellgliedern in Gestalt von Drosselventilen, vorzugsweise einstellbar, die in Dekontaminationsmittel oder Druckluft führenden Stoffleitungen installiert sind.
Die in die Anordnung zugeführte Druckluft und Umgebungsluft durchströmt reinigende Filter.

Die Dosiervorrichtung ist als kompakte Baueinheit beschaffen und lässt sich in unmittelbarer Nähe zum Containment installieren, um eine minimale Länge der Stoffleitung vom Speicherraum zur Sprühdüse und damit eine minimale Transportzeit für das vom Speicherraum an die Sprühdüse in Portionen zugeführte Dekontaminationsmittel zu erzielen. Der Tank, die Quelle für die Druckluft und die Steuereinrichtung befinden sich ausserhalb der Dosiervorrichtung. Hierbei könnte die Steuerung für die Dosiervorrichtung über die ohnehin für das Containment vorhandene zentrale Steuereinrichtung erfolgen. Alternativ könnte man eine separate, in die Dosiervorrichtung integrierte Steuereinrichtung vorsehen.

An der Dosiervorrichtung gemäss einer *ersten Variante* der Anordnung sind definiert:
a) ein erster Anschlusspunkt, durch den eine vom Tank kommende Stoffleitung in die Dosiervorrichtung führt, wobei in den Tank eine Stoffleitung einmündet, die eine Zuführung aus der Umgebungsluft bildet; und
   b) ein zweiter Anschlusspunkt, ein dritter Anschlusspunkt und ein vierter Anschlusspunkt, durch die jeweils eine vom Druckluftanschluss kommende Stoffleitung in die Dosiervorrichtung führt.

Die Dosiervorrichtung umfasst ferner:
a) ein erstes Stellglied, an das die sich vom ersten Anschlusspunkt fortsetzende Stoffleitung führt und das über eine Steuerleitung mit der Steuereinrichtung verbunden ist;
b) ein fünftes Stellglied, an das die sich vom zweiten Anschlusspunkt fortsetzende Stoffleitung führt und das über eine Steuerleitung mit der Steuereinrichtung verbunden ist; und
c) ein sechstes Stellglied, an das die sich vom vierten Anschlusspunkt fortsetzende Stoffleitung führt und das über eine Steuerleitung mit der Steuereinrichtung verbunden ist.
In der zum dritten Anschlusspunkt Druckluft führenden Stoffleitung ist ein viertes Stellglied installiert, das über eine Steuerleitung mit der Steuereinrichtung verbunden ist.

Vom ersten Stellglied setzt sich eine Stoffleitung zum Dosierbehältnis mit dem darin vorhandenen Speicherraum fort, und vom ersten Stellglied erstreckt sich eine weitere Stoffleitung zur Sprühdüse. Vom fünften Stellglied setzt sich eine Stoffleitung fort, die sich zu einem in die Umgebungsluft mündenden Förderer, vorzugsweise in Gestalt einer Venturidüse, erstreckt. Vom dritten Anschlusspunkt erstreckt sich eine Stoffleitung zur Sprühdüse. Vom sechsten Stellglied setzt sich eine Stoffleitung fort, die oberhalb eines Füllstands-Sensors in die zum ersten Sicherungselement weiterführende Stoffleitung mündet.

Vom Speicherraum erstreckt sich eine Stoffleitung über einen via Steuerleitung mit der Steuereinrichtung verbundenen Füllstands-Sensor weiter zu einem ersten Sicherungselement und von hier an den Förderer. In der Stoffleitung ist zwischen dem fünften Stellglied und dem Förderer ein achtes Stellglied eingebaut, vorzugsweise in Gestalt eines einstellbaren Drosselventils. In der Stoffleitung ist zwischen dem sechsten Stellglied und ihrer Einmündung in die zum ersten Sicherungselement weiterführenden Stoffleitung ein neuntes Stellglied installiert, vorzugsweise in Gestalt eines Drosselventils.

In der Stoffleitung sitzt zwischen dem ersten Stellglied und dem Dosierbehältnis ein via Steuerleitung mit der Steuereinrichtung verbundener Leerstands-Sensor. In der Stoffleitung ist zwischen dem ersten Sicherungselement und dem Förderer ein zweites Sicherungselement vorgesehen, wobei die beiden Sicherungselemente vorzugsweise als semipermeable Membranen ausgebildet sind. In der anderen Stoffleitung ist zwischen dem ersten Stellglied und der Sprühdüse ein siebentes Stellglied installiert, vorzugsweise in Gestalt eines einstellbaren Drosselventils.

### Kurzbeschreibung der beigefügten Zeichnungen

Es zeigen:
- Figur 1A -: das Schaltbild einer *ersten Variante* der Anordnung;
- Figur 1B -: die Dosiervorrichtung aus Figur 1A, als perspektivische Prinzipdarstellung;
- Figur 1C -: die Dosiervorrichtung gemäss Figur 1B, in Frontansicht;
- Figur 1D -: die Dosiervorrichtung gemäss Figur 1B, in Seitenansicht;
- Figur 1E -: die Dosiervorrichtung gemäss Figur 1B, in partieller Explosivdarstellung;
- Figur 1F -: die Dosiervorrichtung gemäss Figur 1B, in detaillierterer Explosivdarstellung;
- Figur 1G -: die Dosiervorrichtung gemäss Figur 1B, in noch detaillierterer Explosivdarstellung;
- Figur 1H -: den Vertikalschnitt auf der Linie A-A in Figur 1D;
- Figur 1J -: den Horizontalschnitt auf der Linie B-B in Figur 1D;
- Figur 2A -: das Schaltbild einer *zweiten Variante* der Anordnung;
- Figur 2B -: die Dosiervorrichtung aus Figur 2A, als perspektivische Prinzipdarstellung;
- Figur 2C -: die Dosiervorrichtung gemäss Figur 2B, in partieller Explosivdarstellung;
- Figur 3A -: das Schaltbild einer *dritten Variante* der Anordnung;
- Figur 3B -: die Dosiervorrichtung aus Figur 3A, als perspektivische Prinzipdarstellung;
- Figur 3C -: die Dosiervorrichtung gemäss Figur 3B, in partieller Explosivdarstellung;
- Figur 4A -: das Schaltbild einer *vierten Variante* der Anordnung;
- Figur 4B -: die Dosiervorrichtung aus Figur 4A, als perspektivische Prinzipdarstellung;
- Figur 4C -: die Dosiervorrichtung gemäss Figur 4B, in partieller Explosivdarstellung;
- Figur 5 -: das Schaltbild einer *fünften Variante* der Anordnung;
- Figur 6 -: das Schaltbild einer *sechsten Variante* der Anordnung;
- Figur 7 -: das Schaltbild einer *siebenten Variante* der Anordnung;
- Figur 8 -: das Schaltbild einer *achten Variante* der Anordnung;
- Figur 9A -: das Schaltbild einer *neunten Variante* der Anordnung;
- Figur 9B -: die Dosiervorrichtung aus Figur 9A, mit in der Grösse einstellbarem Speicherraum und angeschlossenem Tank zur Bevorratung von Dekontaminationsmittel, als perspektivische Prinzipdarstellung;
- Figur 9C -: das Dosierbehältnis aus Figur 9B, mit leerem Speicherraum in grosser Volumeneinstellung und einem kugelförmigen Verschlusselement als Auftriebskörper, in Perspektivansicht;
- Figur 9D -: das Dosierbehältnis gemäss Figur 9C, im vergrösserten Vertikalschnitt;
- Figur 9E -: die Darstellung gemäss Figur 9C, mit gefülltem Speicherraum und kleiner Volumeneinstellung;
- Figur 9F -: das Dosierbehältnis gemäss Figur 9E, im vergrösserten Vertikalschnitt;
- Figur 9G -: das Dosierbehältnis aus Figur 9B, mit einem Verschlusselement als semipermeable Membran, im Vertikalschnitt;
- Figur 9H -: ein abgewandeltes Dosierbehältnis, mit elektrisch einstellbarer Portionsgrösse im Speicherraum, in Perspektivansicht;
- Figur 9J -: den Aufbau gemäss Figur 9H, in Draufsicht; und
- Figur 9K -: den Vertikalschnitt auf der Linie C-C in Figur 9J.

### Ausführungsbeispiel

Mit Bezug auf die beiliegenden Zeichnungen erfolgt nachstehend die detaillierte Beschreibung der erfindungsgemässen Anordnung zum Einbringen einer Sollmenge an Dekontaminationsmittel in ein Containment. Hierbei werden der konstruktive Aufbau von insgesamt neun Varianten der Anordnung sowie deren Funktion erörtert. Im Interesse, Wiederholungen zu vermeiden, gilt für die Beschreibung der einzelnen Varianten folgende Festlegung, sind in einer zu einer Variante gehörenden Figurenfolge Bezugsziffern enthalten, aber im zugehörigen Beschreibungstext nicht erläutert, so wird auf deren Erläuterung in vorangehenden Varianten Bezug genommen.

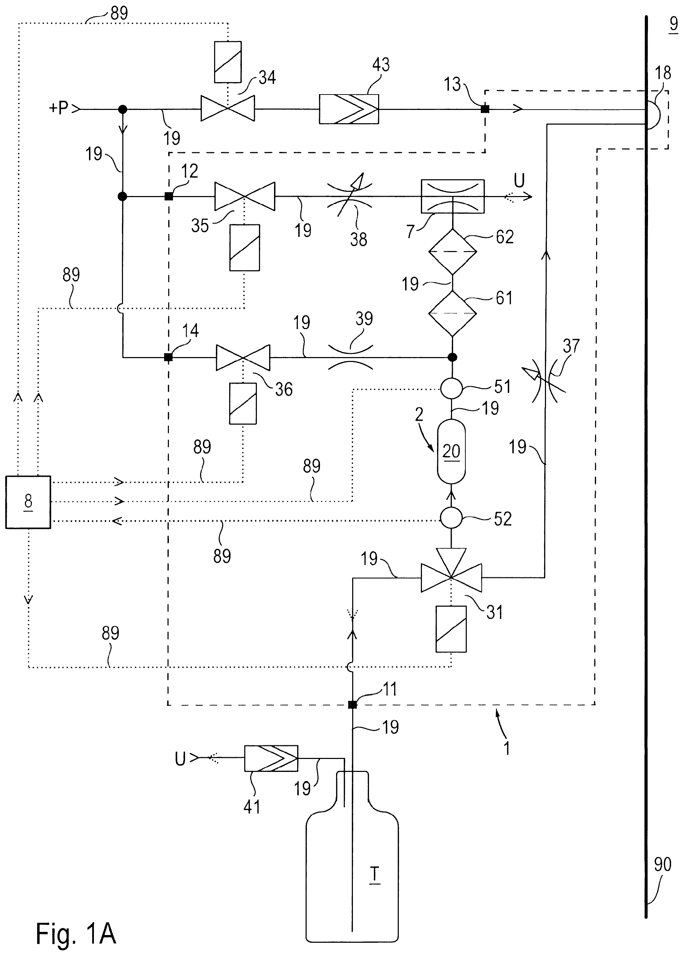

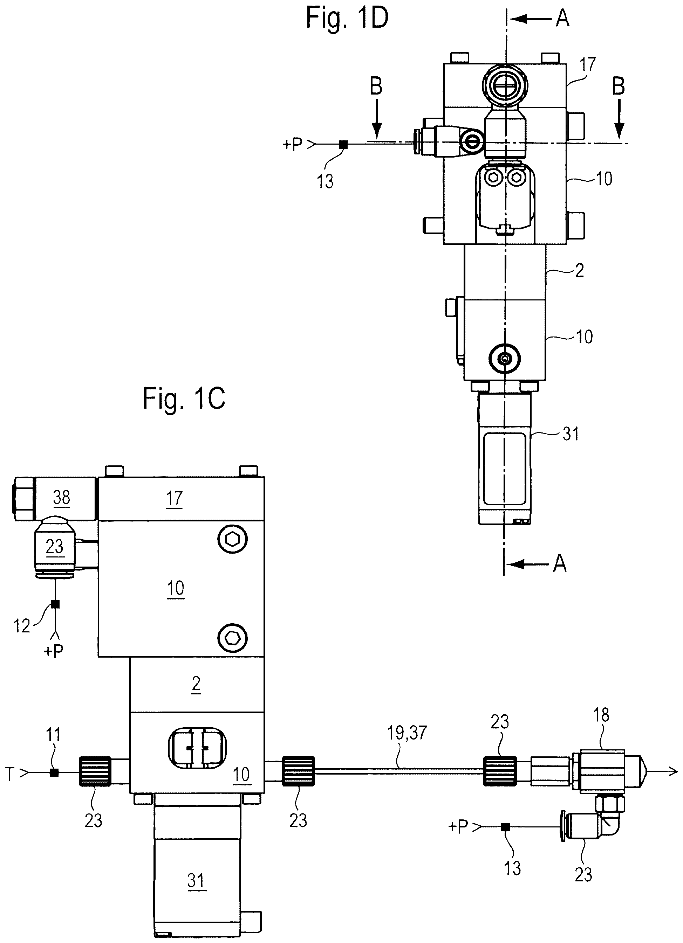

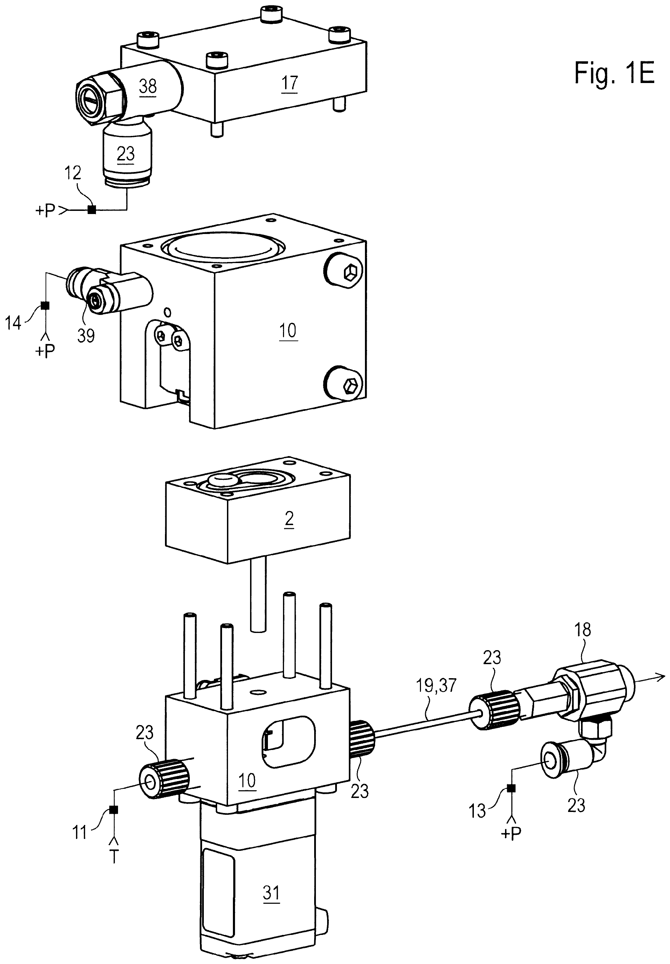

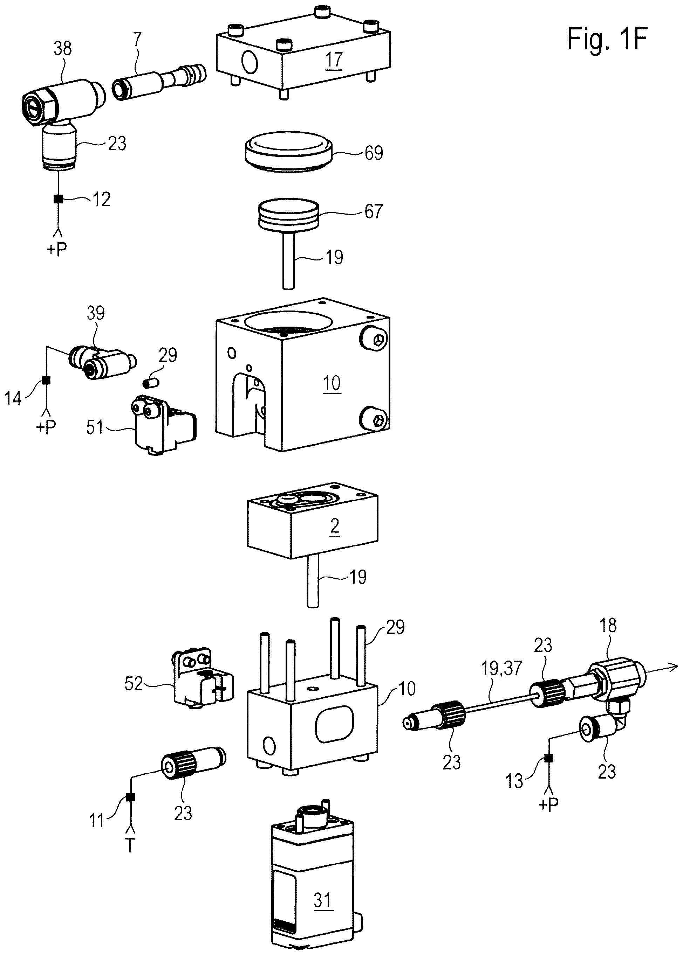

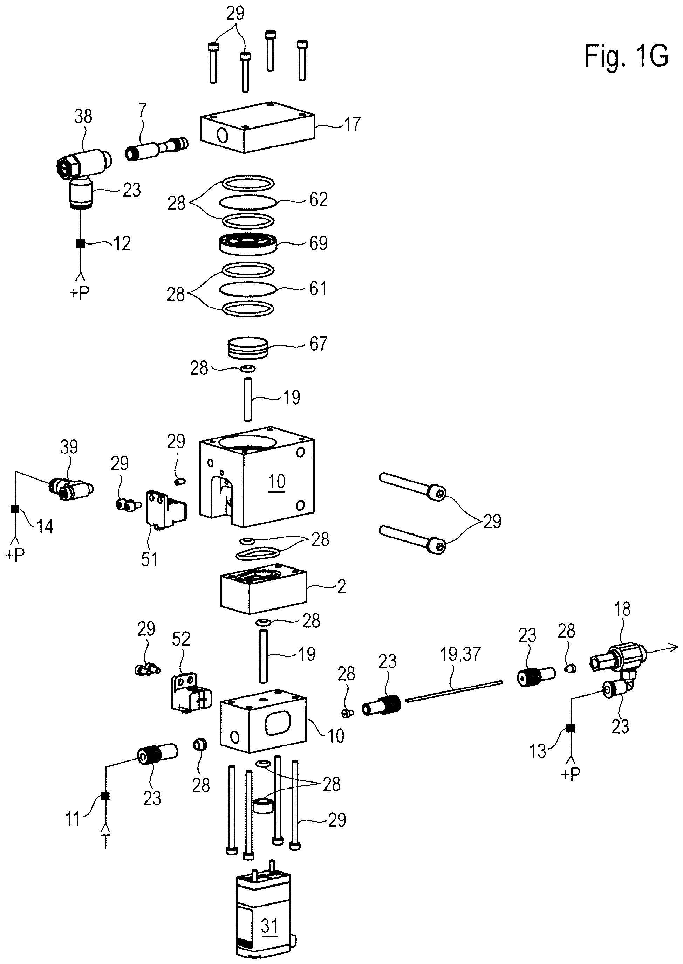

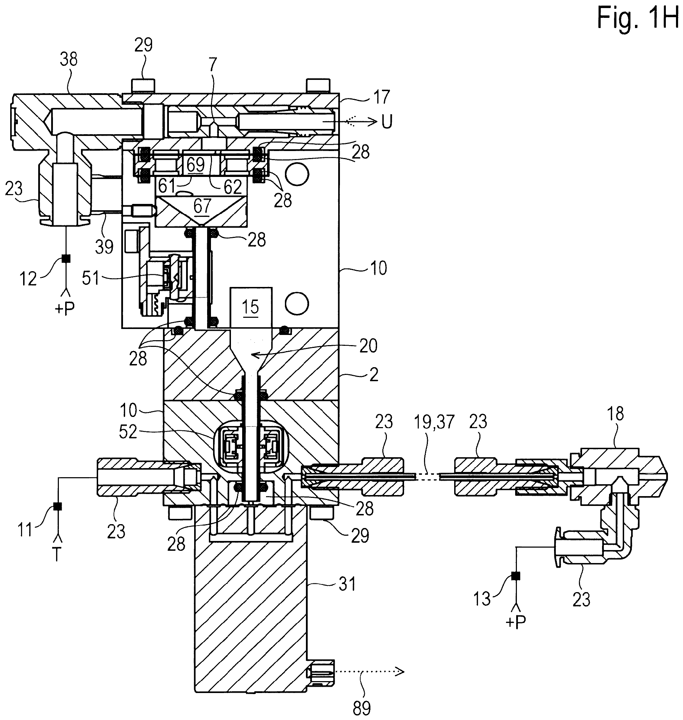

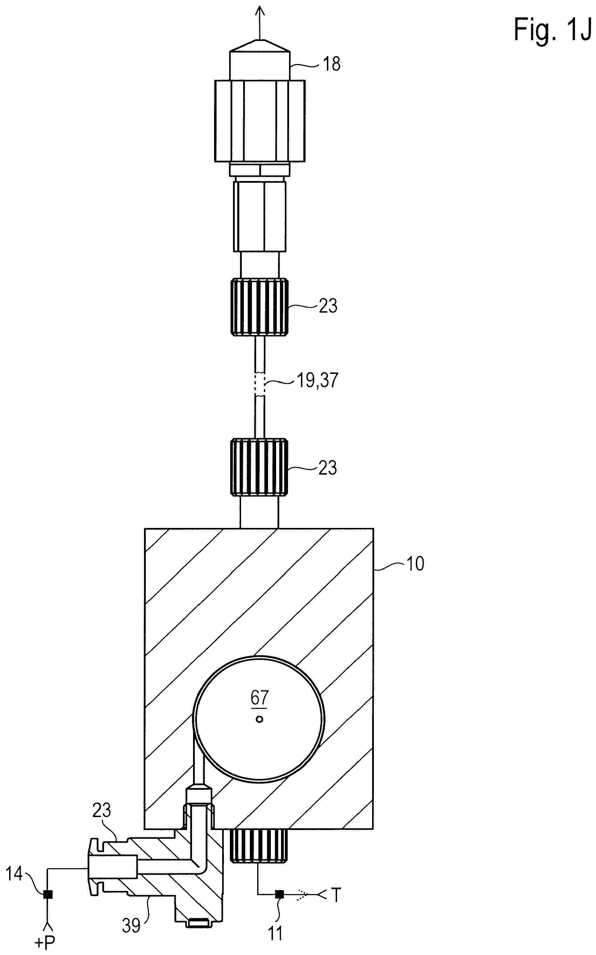

### Figuren 1A bis 1J (erste Variante der Anordnung)

An der Dosiervorrichtung **1** wird zunächst ein erster Anschlusspunkt **11** definiert, durch den eine vom Tank **T** kommende Stoffleitung **19** in die Dosiervorrichtung **1** führt, wobei in den Tank **T** eine Stoffleitung **19** einmündet, die eine Zuführung aus der Umgebungsluft **U** bildet. Vorhanden sind ferner ein zweiter Anschlusspunkt **12,** ein dritter Anschlusspunkt **13** und ein vierter Anschlusspunkt **14,** durch die jeweils eine vom Druckluftanschluss **+P** kommende Stoffleitung **19** in die Dosiervorrichtung **1** führt. An ein erstes Stellglied **31** führt die sich vom ersten Anschlusspunkt **11** fortsetzende Stoffleitung **19** zugleich ist das erste Stellglied **31** über eine Steuerleitung **89** mit der Steuereinrichtung **8** verbunden. An ein fünftes Stellglied **35** führt die sich vom zweiten Anschlusspunkt **12** fortsetzende Stoffleitung **19,** und zugleich ist das fünfte Stellglied **35** über eine Steuerleitung **89** mit der Steuereinrichtung **8** verbunden. An ein sechstes Stellglied **36** führt die sich vom vierten Anschlusspunkt **14** fortsetzende Stoffleitung **19** und zugleich ist das sechste Stellglied **36** über eine Steuerleitung **89** mit der Steuereinrichtung **8** verbunden. In der zum dritten Anschlusspunkt **13** Druckluft **+P** führenden Stoffleitung **19** ist ein viertes Stellglied **34** installiert, das über eine Steuerleitung **89** mit der Steuereinrichtung **8** verbunden ist.

Vom ersten Stellglied **31** setzt sich eine Stoffleitung **19** zum Dosierbehältnis **2** mit dem darin vorhandenen Speicherraum **20** - z.B. in der Grösse von 1 cm³ - fort. Vom ersten Stellglied **31** erstreckt sich eine weitere Stoffleitung **19** zur Sprühdüse **18,** die in das Containment **9** mündend in dessen Kammerwand **90** eingesetzt ist. Vom fünften Stellglied **35** setzt sich eine Stoffleitung **19** fort, die sich zu einem in Umgebungsluft **U** mündenden Förderer **7,** vorzugsweise in Gestalt einer Venturidüse, erstreckt. Vom dritten Anschlusspunkt **13** erstreckt sich eine Stoffleitung **19** zur Sprühdüse **18.** Vom sechsten Stellglied **36** setzt sich eine Stoffleitung **19** fort, die oberhalb eines Füllstands-Sensors **51** in die zum ersten Sicherungselement **61** weiterführende Stoffleitung **19** mündet.

Vom Speicherraum **20** verläuft eine Stoffleitung **19** über einen via Steuerleitung **89** mit der Steuereinrichtung **8** verbundenen Füllstands-Sensor **51** weiter zu einem ersten Sicherungselement **61** und von hier an den Förderer **7.** In der Stoffleitung **19** ist zwischen dem fünften Stellglied **35** und dem Förderer **7** ein achtes Stellglied **38,** vorzugsweise in Gestalt eines einstellbaren Drosselventils, installiert. In der Stoffleitung **19** zwischen dem sechsten Stellglied **36** und ihrer Einmündung in die zum ersten Sicherungselement **61** weiterführenden Stoffleitung **19** ist ein neuntes Stellglied **39,** vorzugsweise in Gestalt eines Drosselventils, installiert. In der Stoffleitung **19** zwischen dem ersten Stellglied **31** und dem Dosierbehältnis **2** ist ein via Steuerleitung **89** mit der Steuereinrichtung **8** verbundener Leerstands-Sensor **52** angeordnet. In der Stoffleitung **19** zwischen dem ersten Sicherungselement **61** und dem Förderer **7** ist ein zweites Sicherungselement **62** eingebaut, wobei die beiden Sicherungselemente **61,62** vorzugsweise als semipermeable Membranen ausgebildet sind. Die beiden Sicherungselemente **61,62** bilden eine doppelte Abschirmung, dass versehentlich kein Dekontaminationsmittel über den Förderer **7** in die Umgebung **U** gelangt, andererseits aber ein Luftsog hin zum Speicherraum **20** bewirkt werden kann.

In der anderen Stoffleitung **19** zwischen dem ersten Stellglied **31** und der Sprühdüse **18** sitzt ein siebentes Stellglied **37,** vorzugsweise in Gestalt eines einstellbaren Drosselventils. Die Stellglieder **37-39** dienen der Feinjustierung der Funktion der gesamten Anordnung. Der Strömungswiderstand des siebenten Stellglieds **37** lässt sich z.B. durch den gewählten Strömungsquerschnitt und Länge der Schlauchverbindung zwischen den Leitungsanschlüssen **23** am Gehäuse **10** und an der Sprühdüse **18** bestimmen. Mittels des siebenten Stellglieds **37** wird die Durchflussrate an Dekontaminationsmittel optimiert, um dessen Austritt aus der Sprühdüse **18** in Form eines möglichst feinen Nebels zu erreichen. Das achte Stellglied **38** dient zur Leistungseinstellung des Förderers **7,** um die vom Tank **T** über die Stoffleitung **19,** durch den Speicherraum **20** bis an den ersten Sensor **51** zu haltende Flüssigkeitssäule aufbauen, einschliesslich den auf dieser Strecke vorhandenen Strömungswiderstand zu überwinden.

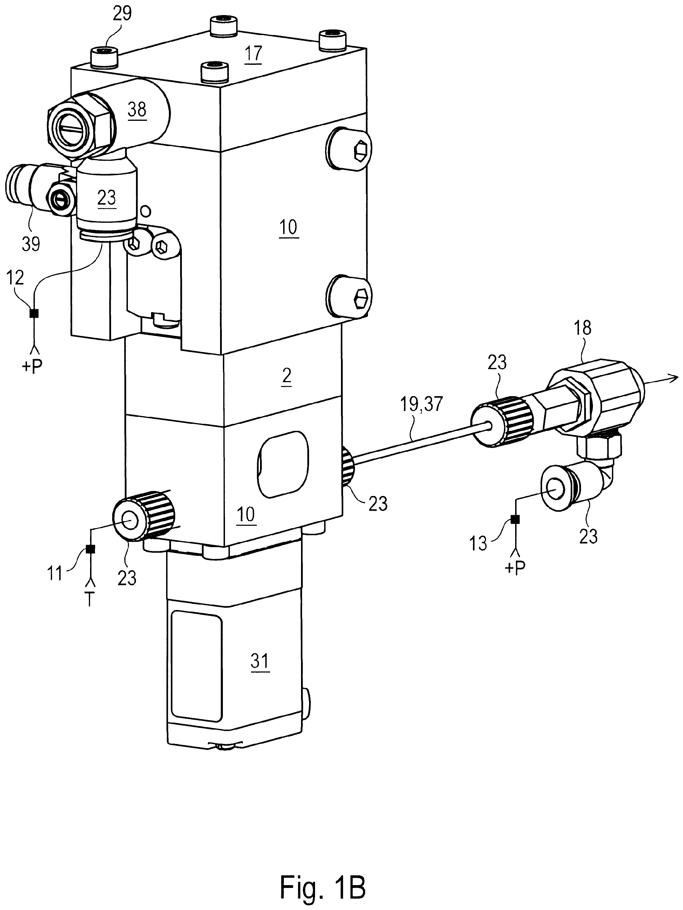

Der Tank **T,** die Steuereinrichtung **8,** die Quelle für die Druckluft **+P** und der Zulauf aus der Umgebungsluft U befinden sich ausserhalb der Dosiervorrichtung **1.** Die innerhalb der Anschlusspunkte **11-14** strukturierte Dosiervorrichtung **1** ist kompakt gestaltet (siehe Figuren 1B bis 1J), gliedert sich bausteinartig in einen Deckel **17,** darunter ein Gehäuse **10,** darunter das Dosierbehältnis **2,** darunter ein weiteres Gehäuse **10** und zuunterst das erste Stellglied **31.** Dichtungen **28** und Schrauben **29** verschiedener Dimensionen dienen zum Zusammenbau der Dosiervorrichtung **1.** Im oberen Gehäuse **10** liegen die beiden von einem Distanzelement **69** auf Abstand gehaltenen Sicherungselemente **62,61,** wobei unterhalb des ersten Sicherungselements **61** ein Trichter **67** angeordnet ist. Oben an den Speicherraum **20** grenzt die Entgasungskammer **15** an, die sich in das obere Gehäuse **10** hinein erstreckt.

Eine von der Umgebungsluft **U** kommende Stoffleitung **19** mündet im Tank **T,** damit beim Absaugen von Dekontaminationsmittel aus dem Tank **T** Luft nachströmen kann, und bei Rückführung von Dekontaminationsmittel aus dem Leitungssystem in den Tank **T** verdrängte Luftmenge in die Umgebungsluft **U** entweichen kann. In dieser Stoffleitung **19** ist ein erster Filter **41** installiert, um zu gewährleisten, dass nur gereinigte Luft in den Tank **T** gelangt.

Beim Anlagenstart erhalten das erste Stellglied **31** und das fünfte Stellglied **35** von der Steuereinrichtung **8** Stellungsimpulse zur Freischaltung des ersten Stellglieds **31** vom Tank **T** hin zum Dosierbehältnis **2** und des fünftes Stellglieds **35** zur Beschickung des Förderers **7** mit Druckluft **+P,** wodurch über die beiden Sicherungselemente **61,62** das Ansaugen von Dekontaminationsmittel in den Speicherraum **20** generiert wird. Beim Erreichen des Sollfüllstands im Speicherraum **20** signalisiert dies der Füllstands-Sensor **51** an die Steuereinrichtung **8,** wodurch das Absaugen aus dem Tank **T** stoppt, eine Umschaltung am ersten Stellglied **31,** die Freischaltung des vierten Stellglieds **34** und das Schliessen des fünften Stellglieds **35** erfolgen. Damit wird die Sprühdüse **18** über die Stoffleitung **19** mit Druckluft **+P** versorgt, der Venturieffekt gestartet und somit die im Speicherraum **20,** in naher Distanz zum Containment **9,** bereitstehende Portion an Dekontaminationsmittel angesaugt und in das Containment **9** in Aerosolform eingebracht. Ein dritter Filter **43** hinter dem vierten Stellglied **34** garantiert, dass nur reine Druckluft **+P** zur Sprühdüse **18** gelangt. Wurde der Speicherraum **20** geleert, signalisiert dies der Leerstands-Sensor **52** an die Steuereinrichtung **8,** und bei Bedarf können die Neubefüllung des Speicherraums **20** mit der nächsten Portion an Dekontaminationsmittel sowie dann deren Verarbeitung gestartet werden.

Ist die Sollmenge an benötigtem Dekontaminationsmittel für die Durchführung einer ordnungsgemässen Dekontamination des Containments **9** mit einer entsprechenden Portionsanzahl **n** erreicht - die Strecke entlang der Stoffleitung **19** vom ersten Stellglied **31** über das siebente Stellglied **37** hin zur Sprühdüse **18** ist leergesaugt - und das Leitungssystem soll entleert werden, erfolgt ein Umschalten in der Steuereinrichtung **8.** Das erste Stellglied **31** öffnet den Rückweg aus dem Speicherraum **20** in den Tank **T.** Die über das sechste Stellglied **36** eingespeiste Druckluft **+P** drückt das noch im Speicherraum **20** sowie in den angrenzenden Stoffleitungen **19** befindliche Dekontaminationsmittel in den Tank **T** zurück. Hierbei dient das neunte Stellglied **39** zur Bemessung der nötigen Intensität der über das sechste Stellglied **36** zugeführten Druckluft **+P** für die Rückführung des restlichen Dekontaminationsmittels in den Tank **T.**

Die Dosiervorrichtung **1** setzt sich im Wesentlichen aus dem oberen und unteren Gehäuseteil **10,** dem dazwischen angeordneten Dosierbehältnis **2** und dem oben aufgesetzten Deckel **17** zusammen. Zur Verbindung mit den jeweiligen Abschnitten an Stoffleitungen **19** sind die Leitungsanschlüsse **23** vorgesehen. Das einstellbare siebente Stellglied **37** wird z.B. durch die gewählte Dimensionierung einer Stoffleitung **19** von bestimmtem Längenabschnitt und Strömungsquerschnitt gebildet.

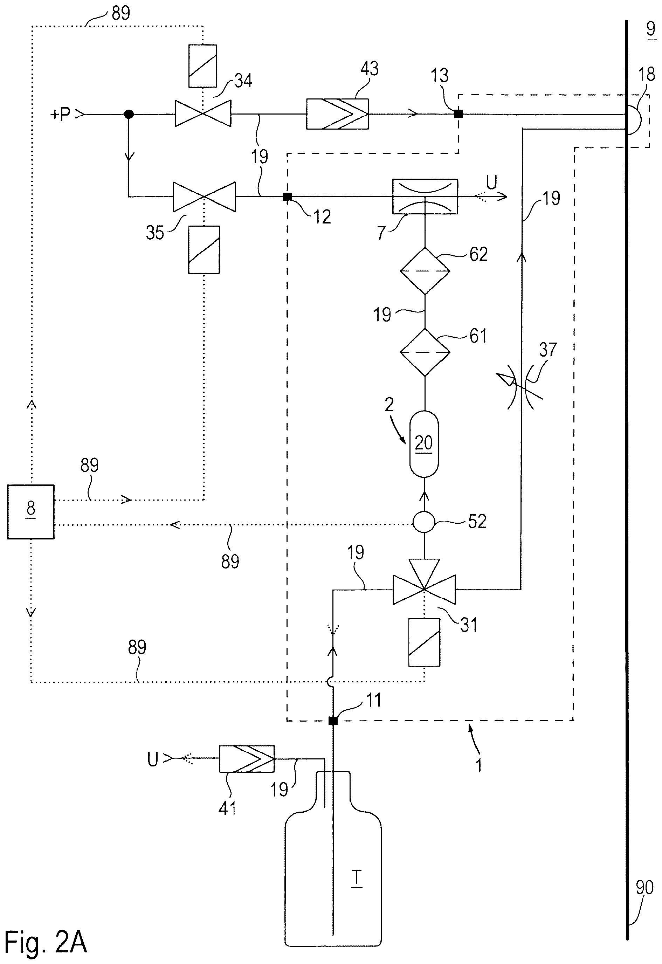

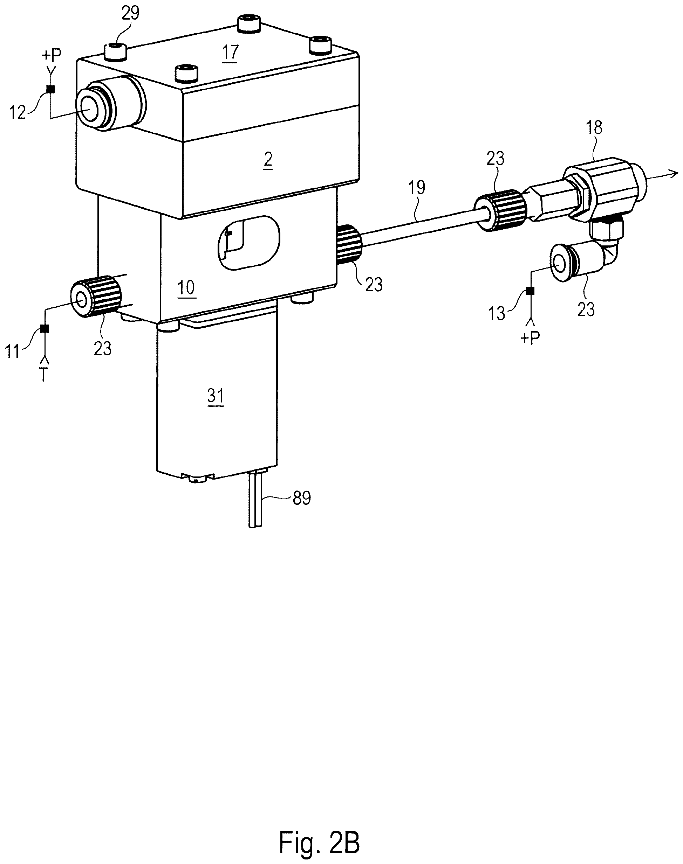

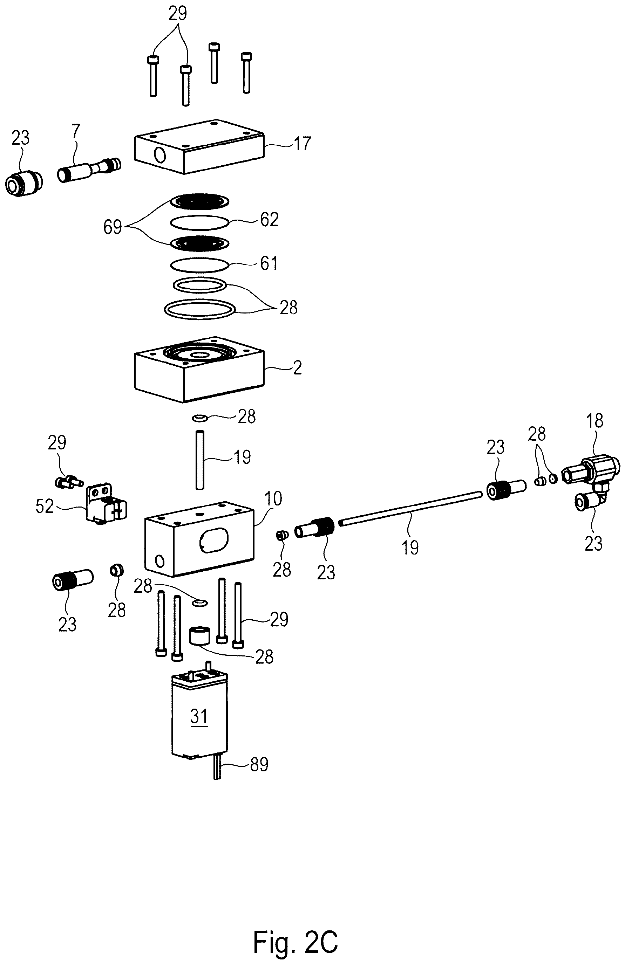

### Figuren 2A bis 2C (zweite Variante der Anordnung)

Diese Dosiervorrichtung **1** setzt sich im Wesentlichen aus dem einen Gehäuseteil **10,** dem darauf angeordneten Dosierbehältnis **2** und dem oben aufgesetzten Deckel **17** zusammen. Gegenüber *erster Variante* hat diese Anordnung einen verminderten apparativen Umfang. Es entfallen der vierte Anschlusspunkt **14,** das sechste Stellglied **36,** das neunte Stellglied **39** und der erste Sensor **51** zur Füllstandssignalisation sowie die zugehörigen Abschnitte an Stoffleitungen **19** und Steuerleitungen **89.** Das Ende der chargenweisen Einspeisung von Dekontaminationsmittel in das Dosierbehältnis **2** erfolgt hier zeitgesteuert, indem das fünfte Stellglied **35** geschlossen wird, worauf das erste Stellglied **31** zur Beschickung der Sprühdüse **18** umschaltet. Während der Entleerung des Dosierbehältnisses **2** - auch bei Rückführung von Dekontaminationsmittel in den Tank **T** - strömt Luft über den Förderer **7** durch die beiden Sicherungselemente **62,61** zum Volumenausgleich in das Dosierbehältnis **2.** Nach Beendigung einer ordnungsgemässen Dekontamination des Containments **9** erfolgt die Rückführung in der Anordnung verbliebenen Dekontaminationsmittels in den Tank **T** nun nicht durch Druck und Sog, sondern allein durch die Schwerkraft aufgrund der Höhendifferenz zum unten positionierten Tank **T.**

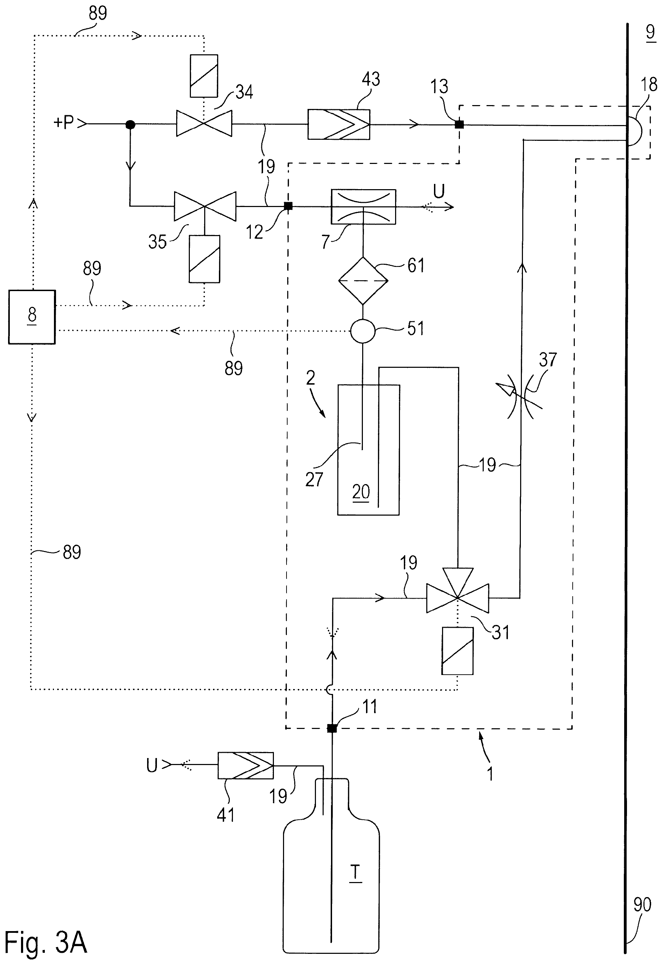

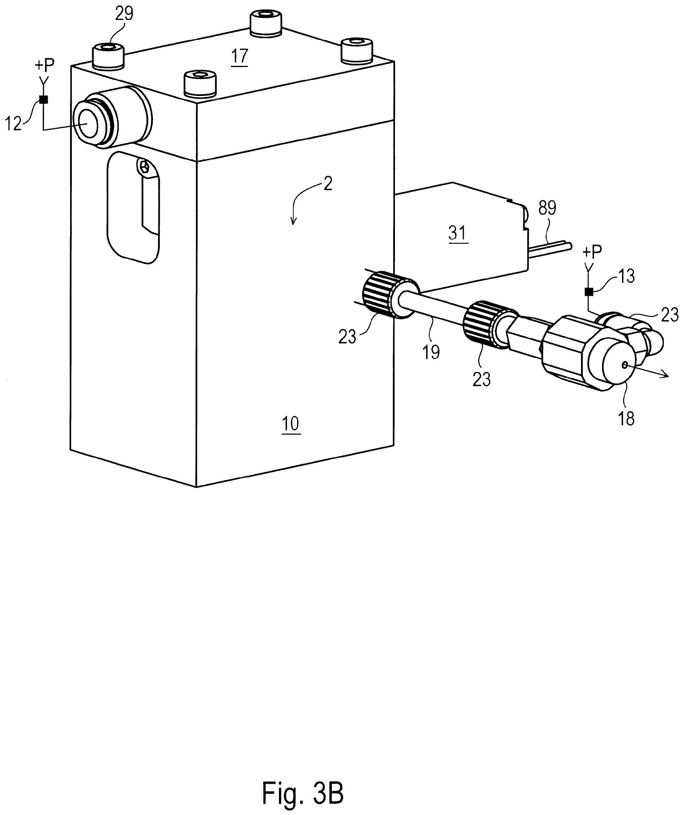

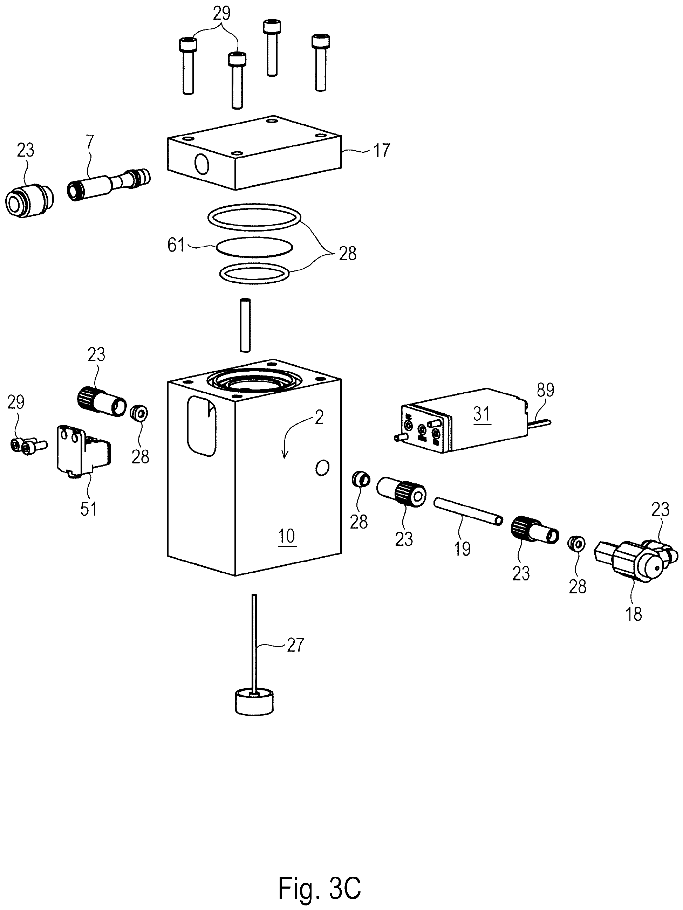

### Figuren 3A bis 3C (dritte Variante der Anordnung)

Diese Dosiervorrichtung **1** ist mit dem Gehäuseteil **10,** dem darin ausgebildeten Dosierbehältnis **2** und dem oben aufgesetzten Deckel **17** noch kompakter. Im Vergleich zur *zweiten Variante* hat man hier auf das zweite Sicherungselement **62** verzichtet, anstelle des zweiten Sensors **52** zur Detektion des Leerstands des Dosierbehältnisses **2** gibt es nun nur den ersten Sensor **51** zur Detektion des Füllstands, und die Grösse des Speicherraums **20** im topfartigen Dosierbehältnis **2** ist jetzt einstellbar ausgebildet, z.B. zwischen 1 cm³ und 50 cm³. Die Einstellbarkeit wird mit einem in den Speicherraum **20** hineinragenden und in der Höhe verschiebbaren Standrohr **27** realisiert.

Der über das geöffnete fünfte Stellglied **35** mit Druckluft **+P** beaufschlagte Förderer **7** bewirkt wiederum das Ansaugen von Dekontaminationsmittel aus dem Tank **T** über das erste Stellglied **31** in das Dosierbehältnis **2,** bis der erste Sensor **51** das Erreichen des eingestellten Füllvolumens anzeigt, worauf das erste Stellglied **31** zur Beschickung der Sprühdüse **18** umschaltet. Die Rückführung in der Anordnung verbliebenen Dekontaminationsmittels in den Tank **T** erfolgt wieder allein durch die Schwerkraftwirkung.

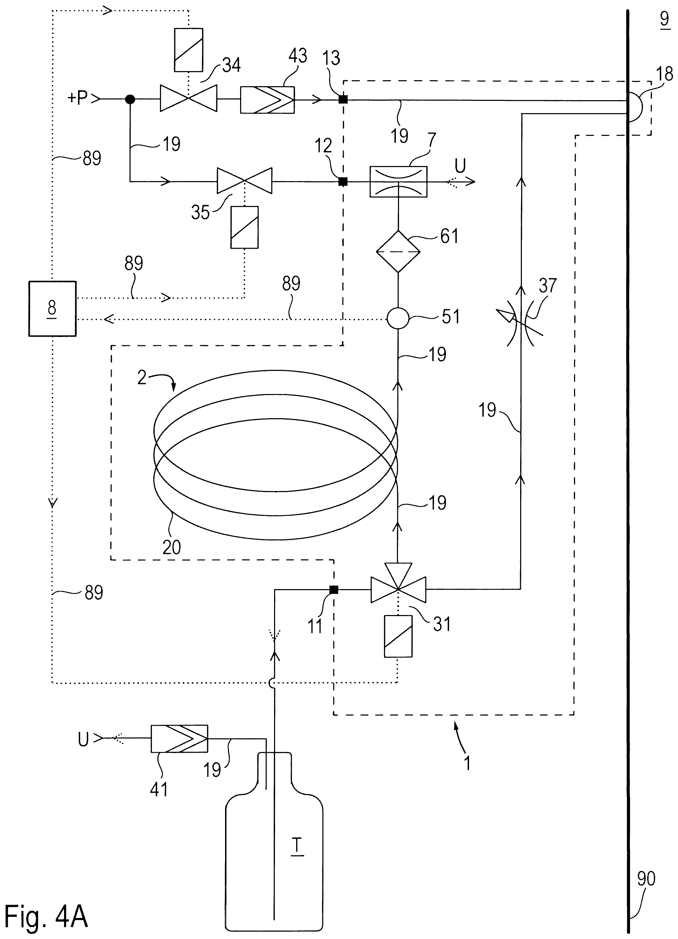

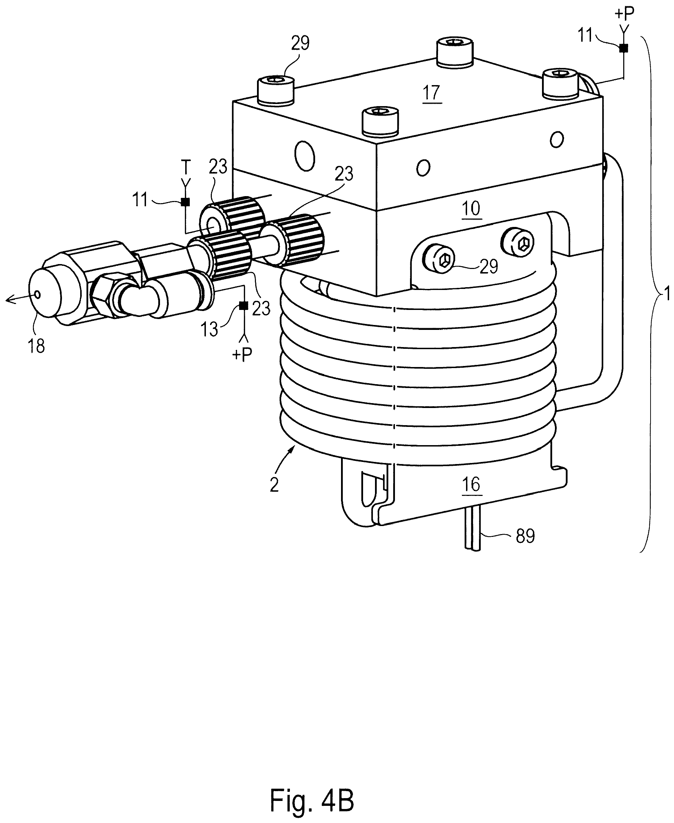

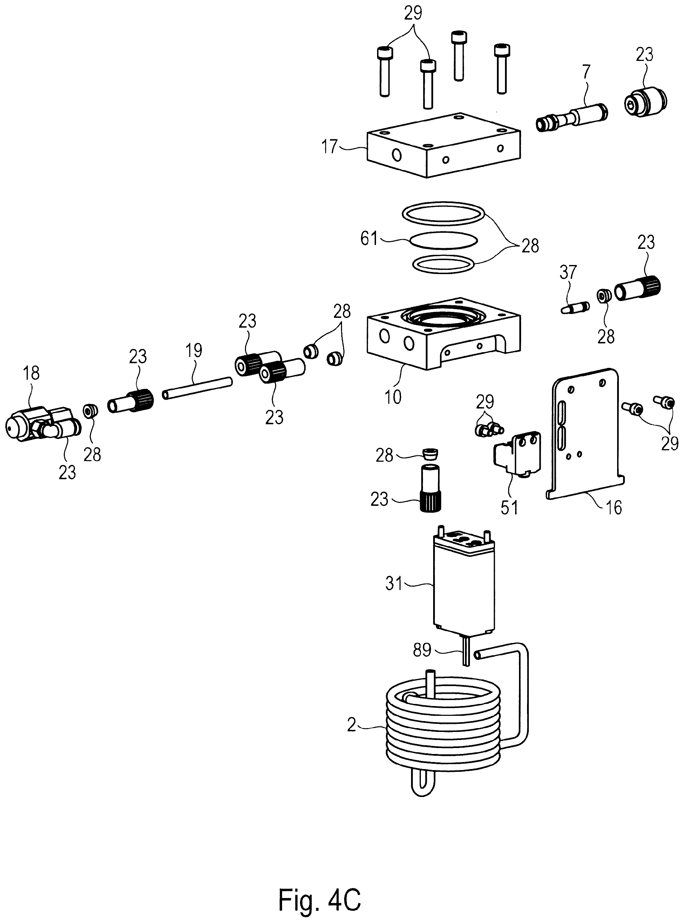

### Figuren 4A bis 4C (vierte Variante der Anordnung)

Auch diese Dosiervorrichtung **1** ist mit dem Gehäuseteil **10,** dem darin ausgebildeten Dosierbehältnis **2** und dem oben aufgesetzten Deckel **17** sehr kompakt gestaltet. Zur Befestigung des ersten Sensors **51** ist zusätzlich eine mit dem Gehäuse **10** zu verschraubende Montageplatte **16** vorgesehen. Der einzige Unterschied zum apparativen Aufbau der *dritten Variante* besteht darin, dass anstelle des zuvor verwendeten Dosierbehältnisses **2** mit dem für das Vorratsvolumen einstellbaren Speicherraum **20** im hiesigen Ausführungsbeispiel die Grösse des Speicherraums **20** durch die Dimensionierung eines Schlauch- oder Rohrwickels definiert wird. Je nach Innenquerschnitt und Länge des Wickels könnte man ein Vorratsvolumen von z.B. zwischen 1 cm³ und 5 cm³ bilden. Befüllen des Dosierbehältnisses **2** mit Dekontaminationsmittel, dessen Eintrag in das Containment **9** über die Sprühdüse **18** und die Rückführung in der Anordnung verbliebenen Dekontaminationsmittels in den Tank **T** geschehen wie bei *dritter Variante.*

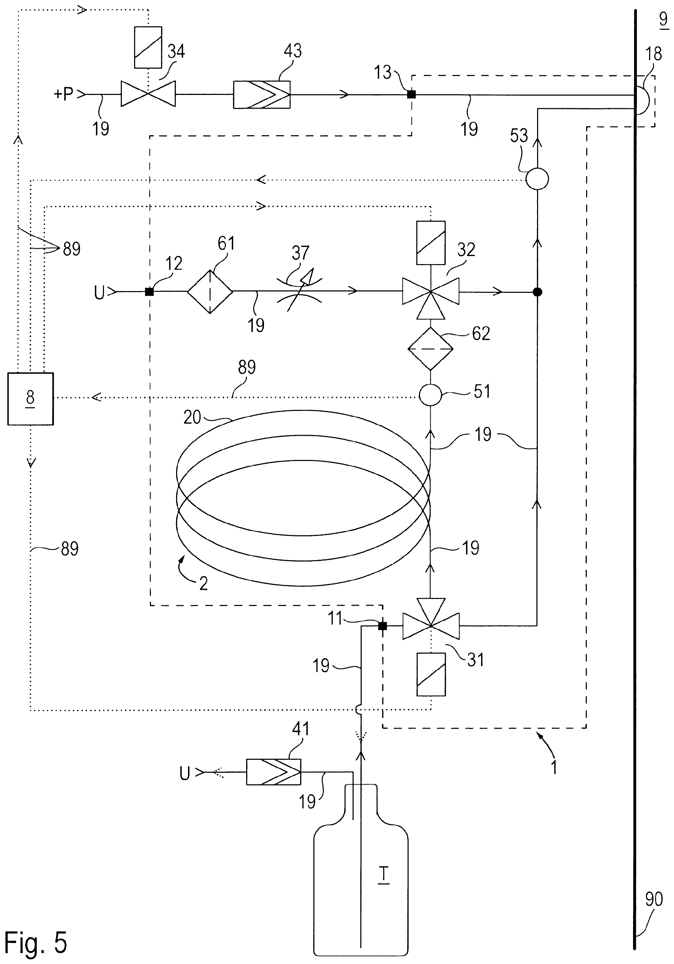

### Figur 5 (fünfte Variante der Anordnung)

In Relation zur *vierten Variante* entfallen das fünfte Stellglied **35** und der Förderer **7,** anstelle dieser Armaturen ist nun ein zweites Stellglied **32** eingebaut. Zum zweiten Stellglied **32** führen von der Steuereinrichtung **8** eine Steuerleitung **89** und aus der Umgebungsluft **U** eine Stoffleitung **19** durch den zweiten Anschlusspunkt **12,** über das erste Sicherungselement **61** und das siebente Stellglied **37.** Mit dem einstellbaren siebenten Stellglied **37** erfolgt die Einstellung der nachströmenden Luft bei der Entleerung des Speicherraums **20.** Zwischen erstem Sensor **51** zur Anzeige des gefüllten Speicherraums **20** und zweitem Stellglied **32** ist jetzt das zweite Sicherungselement **62** positioniert. Ferner ist zwischen der Einmündung der vom zweiten Stellglied **32** abgehenden Stoffleitung **19** in die zur Sprühdüse **18** führenden Stoffleitung **19** ein dritter Sensor **53** installiert, der signalisiert, wenn kein Dekontaminationsmittel ansteht, insbesondere die Portion aus dem Dosierbehältnis **2** über die Sprühdüse **18** verarbeitet ist.

Das Befüllen des Speicherraums **20** mit Dekontaminationsmittel aus dem Tank **T** geschieht jetzt allein durch den Saugeffekt der Sprühdüse **18** über das zweite Stellglied **32** und das zweite Sicherungselement **62.** Das erste Sicherungselement **61** dient als Filter für die aus der Umgebung **U** in die Stoffleitung **19** einströmende Luft und zugleich als Barriere falls Dekontaminationsmittel durch einen Defekt in diesen Abschnitt der Stoffleitung **19** vordringen sollte, welches somit nicht in die Umgebung **U** gelangen kann. Dem ersten Sicherungselement **61** quasi vorgeschaltet ist das zweite Sicherungselement **62.** Die Rückführung in der Anordnung verbliebenen Dekontaminationsmittels in den Tank **T** geschieht auch hier durch die Schwerkraftwirkung.

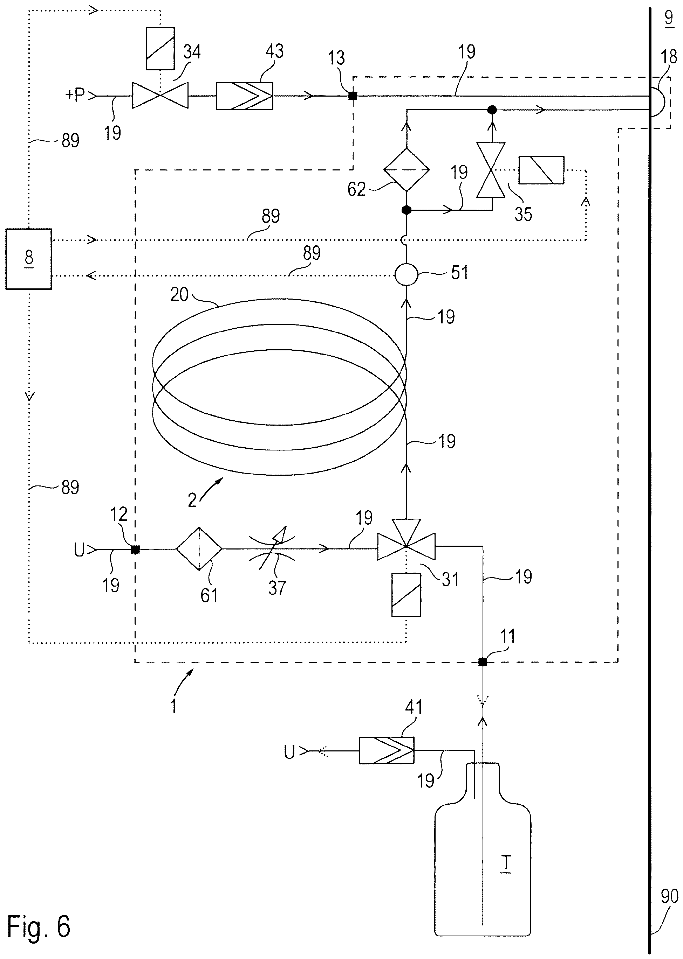

### Figur 6 (sechste Variante der Anordnung)

Bei diesem Ausführungsbeispiel geschieht die Speisung der Sprühdüse **18** mit aus dem Speicherraum **20** abgesaugten Dekontaminationsmittel nicht mehr über das zweite Stellglied **32,** sondern bei Füllstandssignalisation durch den ersten Sensor **51** über das geöffnete fünfte Stellglied **35.** Während der Förderung von Dekontaminationsmittel zur Sprühdüse **18** ist das erste Stellglied **31** mit der Stoffleitung **19** zum Tank **T** hin geschlossen, mit der Stoffleitung **19** zur Umgebungsluft **U** hingegen offen. Aus der Umgebungsluft **U** führt der Abschnitt der Stoffleitung **19** zunächst durch den zweiten Anschlusspunkt **12,** dem das erste Sicherungselement **61** nachgeschaltet ist. Zwischen erstem Sicherungselement **61** und dem Anschluss an das erste Stellglied **31** sitzt in der Stoffleitung **19** das einstellbare siebente Stellglied **37,** wobei erstes Sicherungselement **61** und siebentes Stellglied **37** die Funktion wie zuvor beschrieben haben (siehe Figur 5).

Signalisiert der erste Sensor **51** hingegen nicht das Anstehen von Dekontaminationsmittel, sondern von Luft, wird damit angezeigt, dass der Speicherraum **20** entweder komplett geleert oder noch nicht vollständig gefüllt ist. Folglich werden bzw. bleiben das fünfte Stellglied **35** geschlossen und das erste Stellglied **31** zum Tank **T** hin freigeschaltet, aber zur Umgebungsluft **U** hin geschlossen. Bei fortwährend über das vierte Stellglied **34** eingespeister Druckluft **+P** bewirkt der von der Sprühdüse **18** generierte Ansaugeffekt durch das zweite Sicherungselement **62** und den Speicherraum **20** hindurch dessen erneute bzw. Komplettierung der Füllung mit einer nächsten Portion **n** von Dekontaminationsmittel. Das zweite Sicherungselement **62** lässt jedoch keine eventuell mitgerissenen Partikel von Dekontaminationsmittel hindurch. In der Anordnung verbliebenes Dekontaminationsmittel fliesst wiederum allein durch die Schwerkraftwirkung in den Tank **T** zurück.

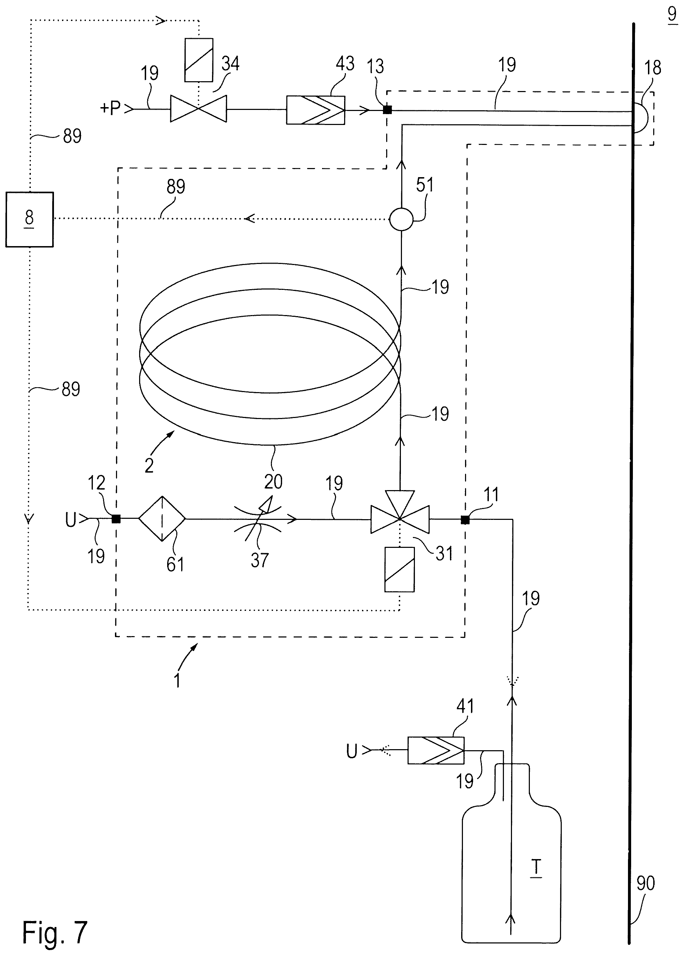

### Figur 7 (siebente Variante der Anordnung)

Dieses Ausführungsbeispiel ist gegenüber der *sechsten Variante* im apparativen Aufbau vereinfacht. Entfallen sind der im Bypass geführte Abschnitt der Stoffleitung **19** mit darin .installiertem fünftem Stellglied **35** und das zweite Sicherungselement **62.** Abgesehen vom geringeren Sicherheitsniveau ist die Funktionsweise weitgehend identisch.

Bei Füllstandssignalisation am ersten Sensor **51** sind, gemäss Impulsen von der Steuereinrichtung **8,** das erste Stellglied **31** zum Tank **T** hin geschlossen und zur Umgebungsluft **U** hin offen. Die über das vierte Stellglied **34** und den dritten Filter **43** mit Druckluft **+P** beaufschlagte Sprühdüse **18** bewirkt das Ansaugen von Dekontaminationsmittel aus dem gefüllten Speicherraum **20** und den Eintrag des Dekontaminationsmittels in zerstäubter Form in das Containment **9.**

Signalisiert der erste Sensor **51** hingegen nur das Anstehen von Luft - der Speicherraum **20** ist also leer bzw. noch nicht komplett gefüllt -, werden bzw. bleiben das erste Stellglied **31** zum Tank **T** hin freigeschaltet und zur Umgebungsluft **U** hin geschlossen. Die über das vierte Stellglied **34** der Sprühdüse **18** zugeleitete Druckluft **+P** erzeugt einen auf den Speicherraum **20** einwirkenden Sog und somit dessen nächste Füllung mit einer weiteren Portion **n** von Dekontaminationsmittel, die dann wieder zum Eintrag in das Containment **9** bereitsteht. Die Rückführung in der Anordnung verbliebenen Dekontaminationsmittels in den Tank **T** basiert auf der Schwerkraftwirkung.

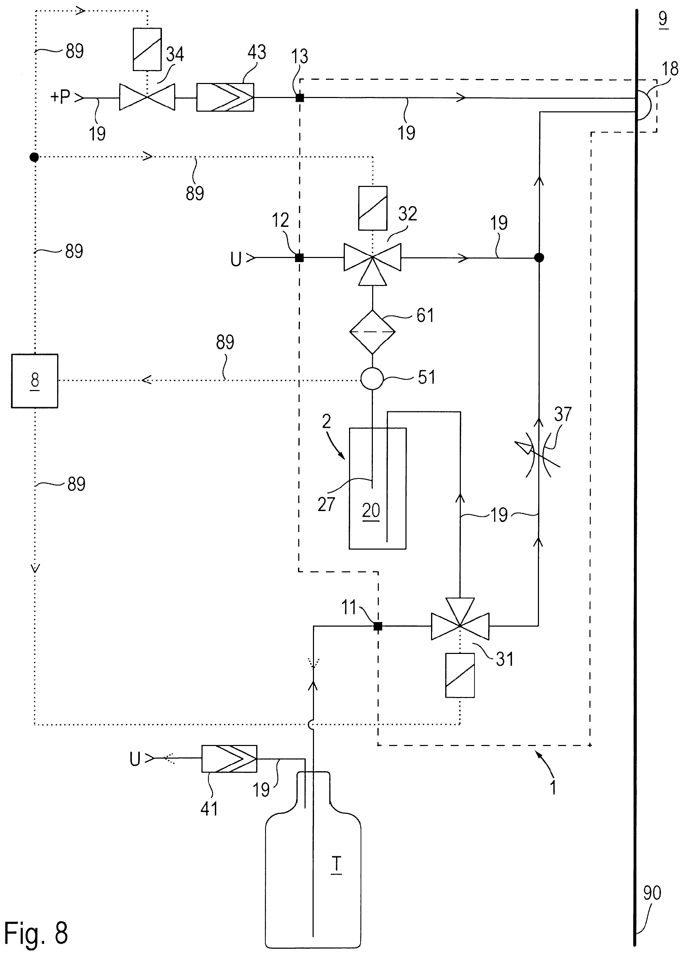

### Figur 8 (achte Variante der Anordnung)

Der Aufbau dieses Ausführungsbeispiels stellt eine nahe Abwandlung zur *fünften Variante* dar. Anstelle des für die Dimensionierung der Grösse des Speicherraums **20** vorgesehenen Schlauch- oder Rohrwickels, kommt nun wieder das in seinem Speichervolumen - z.B. zwischen 1 cm³ und 50 cm³ - einstellbare topfartige Dosierbehältnis **2** zum Einsatz. Nicht vorhanden ist der dritte Sensor **53,** und das erste Sicherungselement **61** sitzt jetzt an der Position des zweiten Sicherungselements **62,** nämlich zwischen erstem Sensor **51** und zweitem Stellglied **32.** Ausserdem befindet sich das einstellbare siebente Stellglied **37** jetzt in der Stoffleitung **19** zwischen erstem Stellglied **31** und der Einmündung der vom zweiten Stellglied **32** in die vom ersten Stellglied **31** kommenden Stoffleitung **19.**

Am Beginn des Füllmodus' erkennt der erste Sensor **51** die unzureichende Füllung des Speicherraums **20.** Die Steuereinrichtung **8** bewirkt die Offenstellung des ersten Stellglieds **31,** nämlich vom Speicherraum **20** nur hin zum Tank **T,** und die Offenstellung des zweiten Stellglieds **32,** nämlich von der Sprühdüse **18** nur hin zum Speicherraum **20,** so dass sich die Sogwirkung von der mit Druckluft **+P** beaufschlagten Sprühdüse **18** über den Speicherraum **20** bis in den Tank **T** erstreckt und der Speicherraum **20** sukzessive mit Dekontaminationsmittel gefüllt wird.

Die vollendete Füllung des Speicherraums **20** wird vom ersten Sensor **51** erkannt und über die Steuereinrichtung **8** verarbeitet, so dass ein Umschalten erfolgt. Das erste Stellglied **31** wechselt in die Offenstellung jetzt nur vom Speicherraum **20** hin zur Sprühdüse **18,** und das zweite Stellglied **32** wechselt in die Offenstellung jetzt von der Umgebungsluft **U** nur mehr hin zum Speicherraum **20.** Damit gelangt entsprechend dem am siebenten Stellglied **37** eingestellten Strömungswiderstand von der Sprühdüse **18** angesaugtes Dekontaminationsmittel mit entsprechender Durchflussrate und in zerstäubter Form in das Containment **9.** In der Anordnung verbliebenes Dekontaminationsmittel fliesst aufgrund Schwerkraftwirkung in den Tank **T** zurück.

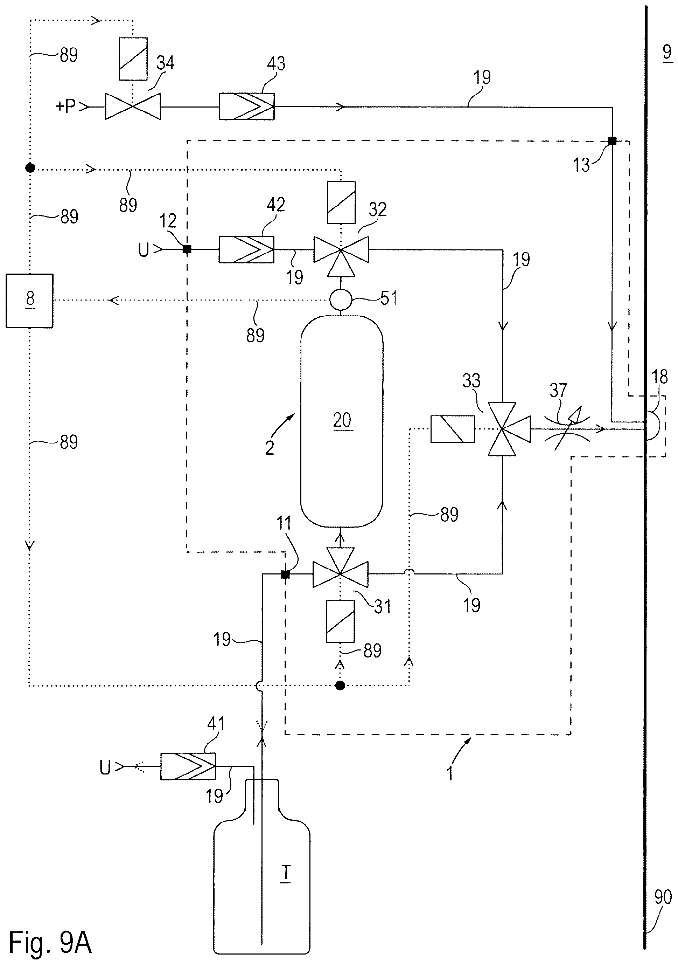

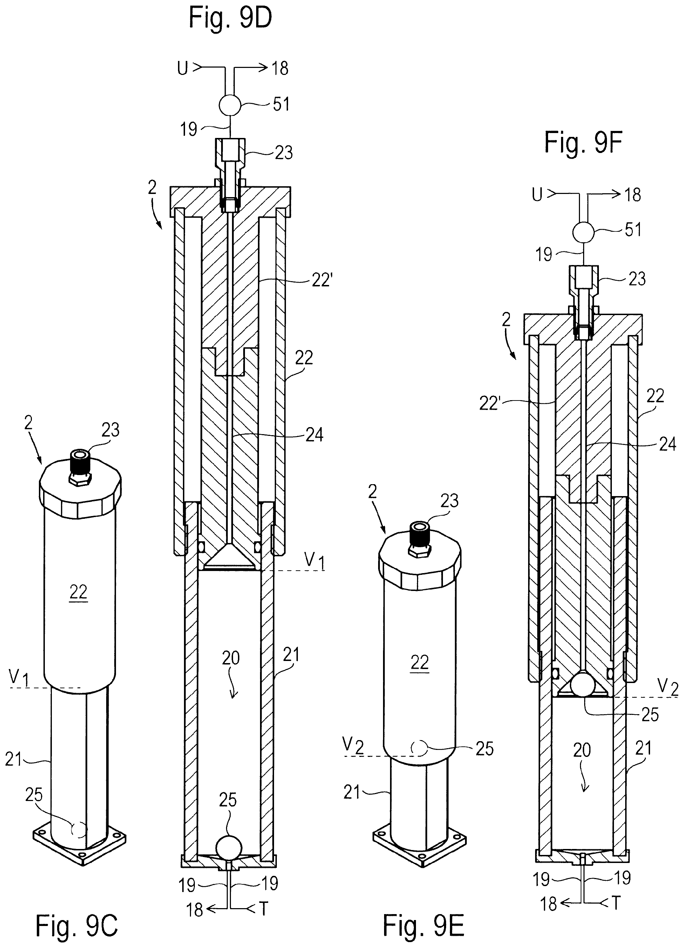

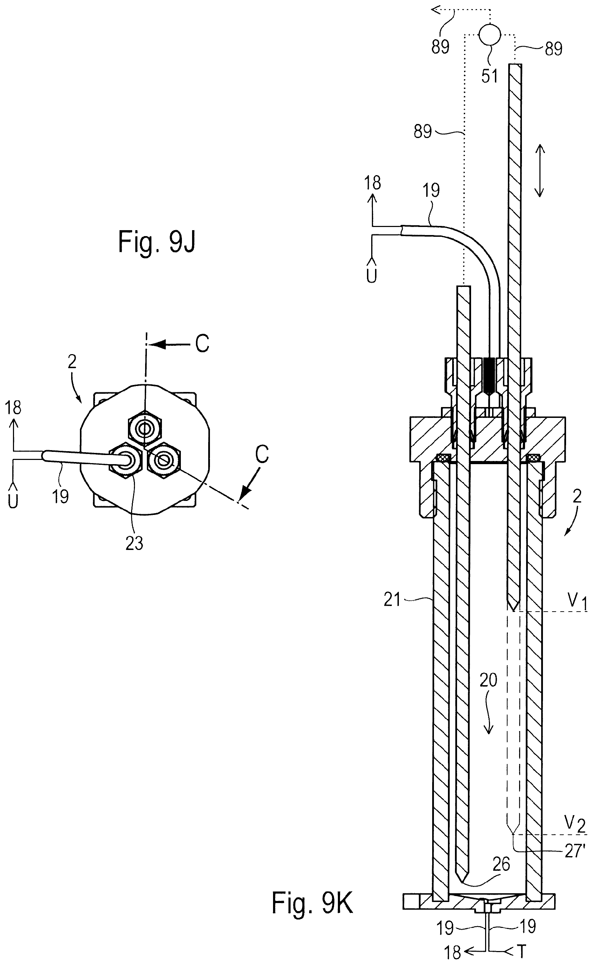

### Figuren 9A bis 9K (neunte Variante der Anordnung)

Für einen Vergleich des apparativen Aufbaus dieser Anordnung wird auf die *achte Variante* Bezug genommen. Der Speicherraum **20** des Dosierbehältnisses **2** ist wiederum einstellbar ausgestaltet, z.B. mit einem Speichervolumen zwischen 1 cm³ und 50 cm³.

Vom Tank **T** erstreckt sich die Stoffleitung **19** durch den ersten Anschlusspunkt **11** zum als 3-Wege-Ventil ausgebildeten ersten Stellglied **31,** von dem ein Anschluss über die Stoffleitung **19** zum Dosierbehältnis **2** und ein weiterer Anschluss über eine Stoffleitung **19** zum dritten Stellglied **33** führt. Vom dritten Stellglied **33** - in Gestalt eines 3-Wege-Ventils - verläuft eine Stoffleitung **19** zum zweiten Stellglied **32,** das ebenfalls ein 3-Wege-Ventil ist, und ein weiterer Anschluss über eine Stoffleitung **19** hin zur Sprühdüse **18.** Zwischen drittem Stellglied 33 und Sprühdüse **18** ist das einstellbare siebente Stellglied **37** angeordnet. Wie bei allen Vorgängervarianten ist von einem Druckluftanschluss **+P** die Stoffleitung **19** durch den dritten Anschlusspunkt **13** an die Sprühdüse **18** herangeführt. Zwischen Druckluftanschluss **+P** und drittem Anschlusspunkt **13** sitzen das vierte Stellglied **34** und der dritte Filter **43** in der Stoffleitung **19.** Ein Anschluss des zweiten Stellglieds **32** mündet mit dem zwischengeschalteten ersten Füllstands-Sensor **51** in das Dosierbehältnis **2** und ein weiterer Anschluss dieses Stellglieds **32** erstreckt sich als Stoffleitung **19** durch den zweiten Anschlusspunkt **12** mit seinem vorgeschalteten zweiten Filter **42** zur Umgebungsluft **U.** Der Füllstands-Sensor **51** und die vier Stellglieder **31-34** sind über Steuerleitungen **89** mit der Steuereinrichtung **8** verbunden.

Beim Auffüllen des Speicherraums **20** steht das erste Stellglied **31** nur vom Dosierbehältnis **2** zum Tank **T** hin in Offenstellung, aber zum dritten Stellglied **33** hin in Schliessstellung. Ein anderer Anschluss des dritten Stellglieds **33** ist zum zweiten Stellglied **32** hin und von dort weiter zum Dosierbehältnis **2** offen. Der verbleibende Anschluss des dritten Stellglieds **33** erstreckt sich offen über das siebente Stellglied **37** an die Sprühdüse **18,** von welcher das Ansaugen des Dekontaminationsmittels generiert wird. Zugleich ist der verbleibende Anschluss des zweiten Stellglieds **33** hin zur Umgebungsluft **U** geschlossen.

Mit dem Erreichen des vom ersten Sensor **51** erkannten eingestellten Füllstands im Speicherraum **20** erfolgt das Umschalten der Anordnung zum Start in den Sprühmodus. Am ersten Stellglied **31** wird die Verbindung vom Dosierbehältnis **2** zum Tank **T** geschlossen und jene zum dritten Stellglied **33** geöffnet. Zugleich wird die Verbindung vom dritten Stellglied **33** hin zum zweiten Stellglied **32** geschlossen und jene vom zweiten Stellglied **32** über den zweiten Anschlusspunkt **12** hin zur Umgebungsluft **U** geöffnet, damit das sukzessive von der mit Druckluft **+P** beaufschlagten Sprühdüse **18** aus dem Speicherraum **20** abgesaugte Dekontaminationsmittel durch nachströmende Luft ersetzt werden kann. In dieser Situation ist die Verbindung, kommend vom Speicherraum **20** über das erste Stellglied **31** und weiter über das dritte Stellglied **33** und das siebente Stellglied **37,** hin zur Sprühdüse **18** offen. Die Rückführung in der Anordnung verbliebenen Dekontaminationsmittels in den Tank **T** geschieht auch hier allein durch Schwerkraftwirkung.

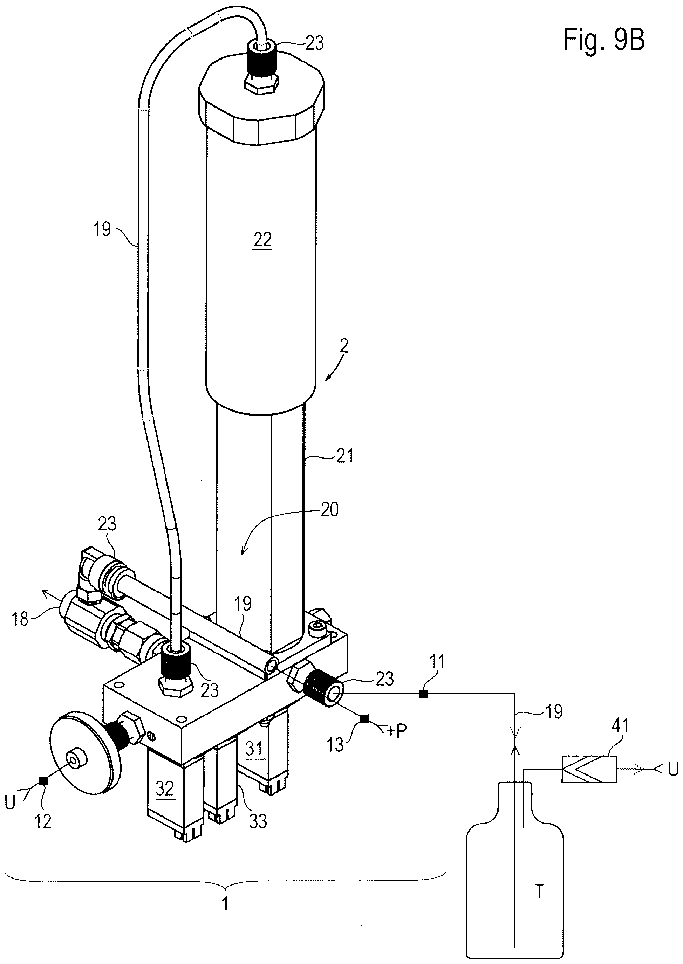

Gemäss den Figuren 9B bis 9G beruht die Einstellbarkeit des Füllvolumens des Speicherraums **20** des Dosierbehältnisses **2** auf einem zylindrischen Körper mit einem unteren Basisteil **21, in** dem sich der Speicherraum **20** befindet, und einem teleskopisch über das Basisteil **21** schiebbaren Hubteil **22.** Das Hubteil **22** besitzt einen axial in den Speicherraum **20** hineinragenden Kolben **22',** der mit zunehmendem Auffahren des Hubteils **22** über das Basisteil **21** die Grösse des Speicherraums **20,** z.B. zwischen einem grösseren Volumen **V₁** und einen kleineren Volumen **V₂** verändert. Im leeren Zustand des Speicherraums **20** liegt ein Verschlusselement **25** - in den Figuren 9C bis 9F eine Schwimmerkugel - am Boden des Basisteils **21.** Mit der sukzessiven Füllung des Speicherraums **20** hebt sich das Verschlusselement **25** auftriebsbedingt bis der Sollstand erreicht ist, bei dem das Verschlusselement **25** die trichterförmige Mündung des sich durch den Kolben **22'** axial erstreckenden Kanals **24** versperrt.

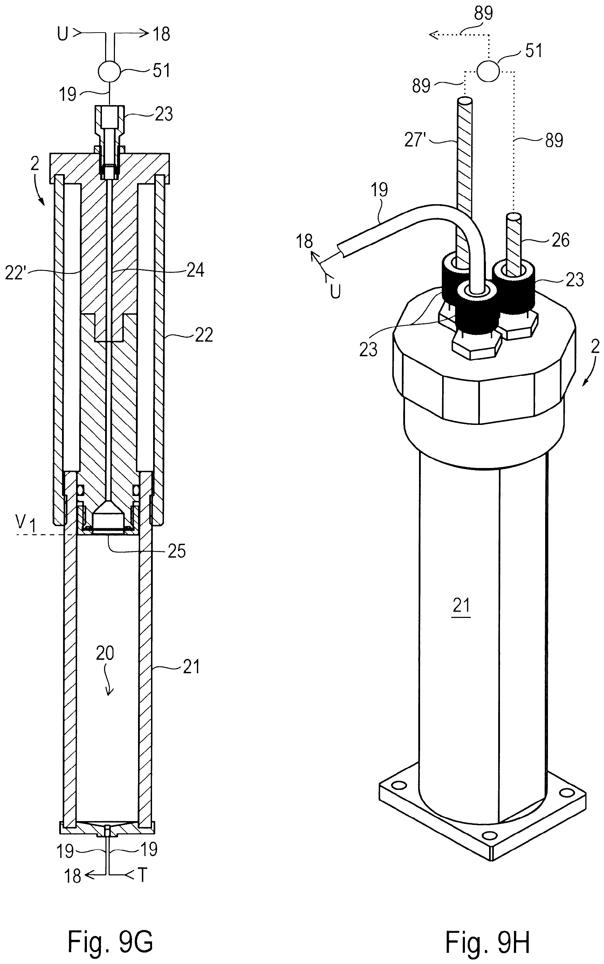

Bei der Ausführungsform des Dosierbehältnisses **2** gemäss Figur 9G ist anstelle des kugelförmigen, auf Auftrieb basierenden Verschlusselements **25** vor der trichterförmigen Mündung des sich durch den Kolben **22'** axial erstreckenden Kanals **24** ein Verschlusselement **25** in Gestalt einer semipermeablen Membran angeordnet, welche kein Dekontaminationsmittel in den Kanal **24** gelangen lässt. Am Basisteil **21** führt eine Stoffleitung **19** vom Tank **T** bzw. von der Sprühdüse **18** in den Speicherraum **20.** Andererseits führt vom Austritt den Kanals **24** ab dem Leitungsanschluss **23** eine Stoffleitung **19** zum Füllstands-Sensor **51** und von dort schliesslich zur Umgebungsluft **U** bzw. der Sprühdüse **18.**

Bei der Ausführungsform des Dosierbehältnisses **2** gemäss den Figuren 9H bis 9K ist nur das Basisteil **21** vorhanden und die Einstellung der Portionsgrösse im Speicherraum **20** geschieht mittels eines in der Einschubtiefe einstellbaren Elektrofühlers **27',** z.B. zwischen einem grösseren Volumen **V₁** und einem kleineren Volumen **V₂.** Bei komplett leerem Speicherraum **20** bzw. noch nicht erreichtem Füllstand werden der im Speicherraum **20** feststehend angeordnete Elektrokontakt **26** und der Elektrofühler **27'** nicht überbrückt, was der erste Sensor **51** erfasst. Hingegen bei erreichtem Füllstand werden Elektrokontakt **26** und Elektrofühler **27'** überbrückt, was der erste Sensor **51** registriert. Die Zuführung in das Basisteil **21** ist äquivalent zu den Figuren 9B bis 9G ausgebildet. Von einem separaten, in den Speicherraum **20** mündenden Leitungsanschluss **23** erstreckt sich eine Stoffleitung **19,** die schliesslich zur Umgebungsluft **U** bzw. der Sprühdüse **18** führt.

## Patentansprüche

1. Anordnung zum Einbringen einer Sollmenge an Dekontaminationsmittel in ein Containment (**9**), umfassend:
a) einen Tank (**T**) als Vorratsbehälter zur Bereitstellung des Dekontaminationsmittels in flüssiger Form;
b) eine Dosiervorrichtung (**1**) mit einer in das Containment (**9**) gerichteten Sprühdüse (**18**) zur Verneblung des Dekontaminationsmittels; und
c) einen Druckluftanschluss (**+P**) sowie eine Steuereinrichtung (**8**) zum Betrieb der Dosiervorrichtung (**1**), **dadurch gekennzeichnet, dass**
d) die Dosiervorrichtung (**1**) ein Dosierbehältnis (**2**) hat, das einen Speicherraum (**20**) mit definiertem Volumen zur Aufnahme einer einzelnen Portion von Dekontaminationsmittel besitzt; und
e) der Speicherraum (**20**) zur taktweisen Füllung mit einer Anzahl (**n**) von Portionen an Dekontaminationsmittel aus dem Tank (**T**) und die im Speicherraum (**20**) jeweils enthaltene Portion zum Einbringen durch die Sprühdüse (**18**) in das Containment (**9**) vor Aufnahme einer nächsten Portion vorgesehen sind, wobei die Portionsanzahl (**n**) zum Erreichen der Sollmenge an benötigtem Dekontaminationsmittel gleich oder grösser 1 wählbar ist.

2. Anordnung nach Anspruch 1, **dadurch gekennzeichnet, dass** der Speicherraum (**20**):
a) mit einer feststehenden oder einstellbaren Grösse ausgebildet ist;
b) als separater Container, Zylinder, Aussparung im Dosierbehältnis (**2**) oder als gestreckte oder geschleifte Rohrlänge beschaffen ist; und
c) der Speicherraum (**20**) ein Volumen im Bereich von 1 cm³ bis 50 cm³, vorzugsweise im Bereich von 1 cm³ bis 5 cm³, hat.

3. Anordnung nach zumindest einem der Ansprüche 1 und 2, **dadurch gekennzeichnet**, zur Einstellbarkeit der Grösse der im Speicherraum (**20**) aufnehmbaren Portion an Dekontaminationsmittel vorgesehen sind:
a) ein Standrohr (**27**), ein Kolben (**22'**) oder ein Elektrofühler (**27'**), die in den Speicherraum (**20**) einschiebbar und in der Position verstellbar sind; oder
b) ein Schlauch- oder Rohrwickel von bestimmtem Innenquerschnitt und Länge des Wickels.

4. Anordnung nach zumindest einem der Ansprüche 1 bis 3, **dadurch gekennzeichnet, dass**
a) der Druckluftanschluss (**+P**) zur Füllung des Speicherraums (**20**) mit Dekontaminationsmittel aus dem Tank (**T**) und zum Betrieb der Sprühdüse (**18**), basierend auf dem Venturi-Prinzip, dient; und
b) zur Füllung des Speicherraums (**20**) mit Dekontaminationsmittel aus dem Tank (**T**) die Dosiervorrichtung (**1**) einen Förderer (**7**) enthält.

5. Anordnung nach zumindest einem der Ansprüche 1 bis 4, **dadurch gekennzeichnet, dass** zur Signalisierung einer in den Speicherraum (**20**) komplett eingefüllten Portion an Dekontaminationsmittel und Stoppen der Förderung aus dem Tank (**T**) ein Füllstands-Sensor (**51**), ein Verschlusselement (**25**) oder ein verstellbares Standrohr (**27**), ein verstellbarer Kolben (**22'**) oder ein verstellbarer Elektrofühler (**27'**) dient.

6. Anordnung nach Anspruch 5, **dadurch gekennzeichnet, dass**
a) das Verschlusselement (**25**) als im Speicherraum (**20**) angeordneter Auftriebskörper oder als semipermeable Membran beschaffen ist; und
b) der verstellbare Elektrofühler (**27'**) mit einem feststehenden Elektrokontakt (**26**) zusammenwirkt, welche bei komplett eingefüllter Portion an Dekontaminationsmittel durch dieses überbrückt sind.

7. Anordnung nach zumindest einem der Ansprüche 1 bis 6, **dadurch gekennzeichnet, dass** an der Steuereinrichtung (**8**) programmierbar sind:
a) der Zeitablauf mit Start, Prozessverlauf und Beendigung für das Einbringen der Sollmenge an Dekontaminationsmittel in das Containment (**9**); und
b) die Sollmenge durch Bestimmung der Portionsanzahl (**n**).

8. Anordnung nach Anspruch 7, **dadurch gekennzeichnet, dass** die Rückführung des in der Dosiervorrichtung (**1**) verbliebenen Dekontaminationsmittels in den Tank (**T**) nach Beendigung des Einbringens der Sollmenge an Dekontaminationsmittel in das Containment (**9**) an der Steuereinrichtung (**8**) programmierbar ist.

9. Anordnung nach zumindest einem der Ansprüche 1 bis 8, **dadurch gekennzeichnet, dass**
a) für das Einbringen der Sollmenge an Dekontaminationsmittel in das Containment (**9**) zur Schaltung des Prozessverlaufs und Mengenregulierung vorgesehen sind:
aa) eine von der Steuereinrichtung (**8**) über Steuerleitungen (**89**) beaufschlagte erste Kategorie von Stellgliedern (**31-33**) in Gestalt von 3-Wege-Ventilen, die in Dekontaminationsmittel oder Umgebungsluft (**U**) führenden Stoffleitungen (**19**) installiert sind;
ab) eine von der Steuereinrichtung (**8**) über Steuerleitungen (**89**) beaufschlagte zweite Kategorie von Stellgliedern (**34-36**) in Gestalt von Absperrventilen, die in Dekontaminationsmittel oder Druckluft (**+P**) führenden Stoffleitungen (**19**) installiert sind;
ac) eine dritte Kategorie von Stellgliedern (**37-39**) in Gestalt von Drosselventilen, vorzugsweise einstellbar, die in Dekontaminationsmittel oder Druckluft (**+P**) führenden Stoffleitungen (**19**) installiert sind; und
b) die in die Anordnung zugeführte Druckluft (**+P**) und Umgebungsluft (**U**) reinigende Filter (**41-43**) durchströmt.

10. Anordnung zumindest einem der Ansprüche 1 bis 9, **dadurch gekennzeichnet, dass**
a) die Dosiervorrichtung (**1**) als kompakte Baueinheit beschaffen ist und sich in unmittelbarer Nähe zum Containment (**9**) installieren lässt, um eine minimale Länge der Stoffleitung (**19**) vom Speicherraum (**20**) zur Sprühdüse (**18**) und damit eine minimale Transportzeit für das vom Speicherraum (**20**) an die Sprühdüse (**18**) in Portionen zugeführte Dekontaminationsmittel zu erzielen; und
b) der Tank (**T**), die Quelle für die Druckluft (**+P**) und die Steuereinrichtung (**8**) sich ausserhalb der Dosiervorrichtung (**1**) befinden; wobei
c) als Steuereinrichtung (**8**) für die Dosiervorrichtung (**1**) eine ohnehin für das Containment (**9**) vorhandene zentrale Steuereinrichtung nutzbar ist oder alternativ eine separate, in die Dosiervorrichtung (**1**) integrierte Steuereinrichtung (**8**) vorsehen werden kann.

11. Anordnung nach zumindest einem der Ansprüche 1 bis 10, **dadurch gekennzeichnet, dass** an der Dosiervorrichtung (**1**) definiert sind:
a) ein erster Anschlusspunkt (**11**), durch den eine vom Tank (**T**) kommende Stoffleitung (**19**) in die Dosiervorrichtung (**1**) führt, wobei in den Tank (**T**) eine Stoffleitung (**19**) einmündet, die eine Zuführung aus der Umgebungsluft (**U**) bildet; und
b) ein zweiter Anschlusspunkt (**12**), ein dritter Anschlusspunkt (**13**) und ein vierter Anschlusspunkt (**14**), durch die jeweils eine vom Druckluftanschluss (**+P**) kommende Stoffleitung (**19**) in die Dosiervorrichtung (**1**) führt.

12. Anordnung nach Anspruch 11, **dadurch gekennzeichnet, dass**
a) die Dosiervorrichtung (**1**) ferner umfasst:
aa) ein erstes Stellglied (**31**), an das die sich vom ersten Anschlusspunkt (**11**) fortsetzende Stoffleitung (**19**) führt und das über eine Steuerleitung (**89**) mit der Steuereinrichtung (**8**) verbunden ist;
ab) ein fünftes Stellglied (**35**), an das die sich vom zweiten Anschlusspunkt (**12**) fortsetzende Stoffleitung (**19**) führt und das über eine Steuerleitung (**89**) mit der Steuereinrichtung (**8**) verbunden ist; und
ac) ein sechstes Stellglied (**36**), an das die sich vom vierten Anschlusspunkt (**14**) fortsetzende Stoffleitung (**19**) führt und das über eine Steuerleitung (**89**) mit der Steuereinrichtung (**8**) verbunden ist; und
b) in der zum dritten Anschlusspunkt (**13**) Druckluft (**+P**) führenden Stoffleitung (**19**) ein viertes Stellglied (**34**) installiert ist, das über eine Steuerleitung (89) mit der Steuereinrichtung (**8**) verbunden ist.

13. Anordnung nach Anspruch 12, **dadurch gekennzeichnet, dass**
a) vom ersten Stellglied (**31**) sich eine Stoffleitung (**19**) zum Dosierbehältnis (**2**) mit dem darin vorhandenen Speicherraum (**20**) fortsetzt, und sich vom ersten Stellglied (**31**) eine weitere Stoffleitung (**19**) zur Sprühdüse (**18**) erstreckt;
b) vom fünften Stellglied (**35**) sich eine Stoffleitung (**19**) fortsetzt, die sich zu einem in die Umgebungsluft (**U**) mündenden Förderer (**7**), vorzugsweise in Gestalt einer Venturidüse, erstreckt;
c) vom dritten Anschlusspunkt (**13**) sich eine Stoffleitung (**19**) zur Sprühdüse (**18**) erstreckt; und
d) vom sechsten Stellglied (**36**) sich eine Stoffleitung (**19**) fortsetzt, die oberhalb eines Füllstands-Sensors (**51**) in die zum ersten Sicherungselement (**61**) weiterführende Stoffleitung (**19**) mündet.

14. Anordnung nach Anspruch 13, **dadurch gekennzeichnet, dass**
a) vom Speicherraum (**20**) sich eine Stoffleitung (**19**) über einen via Steuerleitung (**89**) mit der Steuereinrichtung (**8**) verbundenen Füllstands-Sensor (**51**) weiter zu einem ersten Sicherungselement (**61**) und von hier an den Förderer (**7**) erstreckt;
b) in der Stoffleitung (**19**) zwischen dem fünften Stellglied (**35**) und dem Förderer (**7**) ein achtes Stellglied (**38**), vorzugsweise in Gestalt eines einstellbaren Drosselventils, installiert ist; und
c) in der Stoffleitung (**19**) zwischen dem sechsten Stellglied (**36**) und ihrer Einmündung in die zum ersten Sicherungselement (**61**) weiterführenden Stoffleitung (**19**) ein neuntes Stellglied (**39**), vorzugsweise in Gestalt eines Drosselventils, eingebaut ist.

15. Anordnung nach Anspruch 14, **dadurch gekennzeichnet, dass**
a) in der Stoffleitung (**19**) zwischen dem ersten Stellglied (**31**) und dem Dosierbehältnis (**2**) ein via Steuerleitung (**89**) mit der Steuereinrichtung (**8**) verbundener Leerstands-Sensor (**52**) sitzt;
b) in der Stoffleitung (**19**) zwischen dem ersten Sicherungselement (**61**) und dem Förderer (**7**) ein zweites Sicherungselement (**62**) installiert ist, wobei die beiden Sicherungselemente (**61,62**) vorzugsweise als semipermeable Membranen ausgebildet sind; und
c) in der anderen Stoffleitung (**19**) zwischen dem ersten Stellglied (**31**) und der Sprühdüse (**18**) ein siebentes Stellglied (**37**), vorzugsweise in Gestalt eines einstellbaren Drosselventils, vorgesehen ist.
